# EUROPEAN PATENT SPECIFICATION

(11) **EP 2 043 681 B1**
(45) Date of publication and mention of the grant of the patent: **07.10.2015**
(21) Application number: 07852387.5
(22) Date of filing: 16.07.2007
(51) Int. Cl.: A61K 39/12, C12N 7/02

(54) **CONSTRUCTION OF RECOMBINANT VIRUS VACCINES BY DIRECT TRANSPOSON-MEDIATED INSERTION OF FOREIGN IMMUNOLOGIC DETERMINANTS INTO VECTOR VIRUS PROTEINS**
KONSTRUKTION REKOMBINANTER VIRENIMPFSTOFFE DURCH DIREKTEN TRANSPOSON-VERMITTELTEN EINSATZ FREMDER IMMUNOLOGISCHER DETERMINANTEN IN VEKTORVIRUSPROTEINE
CONSTRUCTION DE VACCINS ANTIVIRAUX DE RECOMBINAISON PAR INSERTION DIRECTE A MEDIATION PAR TRANSPOSON DE DETERMINANTS IMMUNOLOGIQUES ETRANGERS DANS DES PROTEINES DE VIRUS VECTEUR

(30) Priority: 14.07.2006 US 831013 P; 12.01.2007 US 880353 P
(43) Date of publication of application: 08.04.2009
(73) Proprietor: Sanofi Pasteur Biologics, LLC, Cambridge, MA 02139 (US)
(72) Inventor: PUGACHEV, Konstantin, V., Natick, MA 01760 (US); RUMYANTSEV, Alexander, A., Somerville, MA 02144 (US)
(74) Representative: Schnappauf, Georg
(86) International application number: PCT/US2007/016078
(87) International publication number: WO 2008/036146

(56) References cited:
- WO-A-02/072835
- WO-A-02/102828
- WO-A1-98/37911
- ZOU P ET AL: "The epitope recognized by a monoclonal antibody in influenza A virus M2 protein is immunogenic and confers immune protection" INTERNATIONAL IMMUNOPHARMACOLOGY, ELSEVIER, AMSTERDAM, NL, vol. 5, no. 4, 1 April 2005 (2005-04-01), pages 631-635, XP004749880 ISSN: 1567-5769
- BIANCHI ELISABETTA ET AL: "Universal influenza B vaccine based on the maturational cleavage site of the hemagglutinin precursor" JOURNAL OF VIROLOGY, THE AMERICAN SOCIETY FOR MICROBIOLOGY, US, vol. 79, no. 12, 1 June 2005 (2005-06-01), pages 7380-7388, XP002445847 ISSN: 0022-538X
- BARBA-SPAETH GIOVANNA ET AL: "Live attenuated yellow fever 17D infects human DCs and allows for presentation of endogenous and recombinant T cell epitopes." THE JOURNAL OF EXPERIMENTAL MEDICINE 7 NOV 2005, vol. 202, no. 9, 7 November 2005 (2005-11-07), pages 1179-1184, XP002548467 ISSN: 0022-1007

## Description

### Field of the Invention

This invention relates to the construction of recombinant virus vaccines by direct transposon-mediated insertion of foreign immunologic determinants into vector virus proteins and corresponding compositions and methods.

### Background of the Invention

Vaccination is one of the greatest achievements of medicine, and has spared millions of people the effects of devastating diseases. Before vaccines became widely used, infectious diseases killed thousands of children and adults each year in the United States alone, and so many more worldwide. Vaccination is widely used to prevent or treat infection by bacteria, viruses, and other pathogens. Several different approaches are used in vaccination, including the administration of killed pathogen, live-attenuated pathogen, and inactive pathogen subunits. In the case of viral infection, live vaccines have been found to confer the most potent and durable protective immune responses.

Live-attenuated vaccines have been developed against flaviviruses, which are small, enveloped, positive-strand RNA viruses that are generally transmitted by infected mosquitoes and ticks. The *Flavivirus* genus of the *Flaviviridae* family includes approximately 70 viruses, many of which, such as yellow fever (YF), dengue (DEN), Japanese encephalitis (JE), and tick-borne encephalitis (TBE) viruses, are major human pathogens (rev. in Burke and Monath, Fields Virology, 4th Ed.:1043-1126, 2001).

Different approaches have been used in the development of vaccines against flaviviruses. In the case of yellow fever virus, for example, two vaccines (yellow fever 17D and the French neurotropic vaccine) have been developed by serial passage (Monath, "Yellow Fever," In Plotkin and Orenstein, Vaccines, 3rd ed., Saunders, Philadelphia, pp. 815-879, 1999). Another approach to attenuation of flaviviruses for use in vaccination involves the construction of chimeric flaviviruses, which include components of two (or more) different flaviviruses. Understanding how such chimeras are constructed requires an explanation of the structure of the flavivirus genome.

Flavivirus proteins are produced by translation of a single, long open reading frame to generate a polyprotein, which is followed by a complex series of post-translational proteolytic cleavages of the polyprotein by a combination of host and viral proteases to generate mature viral proteins (Amberg et al., J. Virol. 73:8083-8094, 1999; Rice, "Flaviviridae," In Virology, Fields (ed.), Raven-Lippincott, New York, 1995, Volume I, p. 937). The virus structural proteins are arranged in the polyprotein in the order C-prM-E, where "C" is capsid, "prM" is a precursor of the viral envelope-bound membrane (M) protein, and "E" is the envelope protein. These proteins are present in the N-terminal region of the polyprotein, while the non-structural proteins (NS1, NS2A, NS2B, NS3, NS4A, NS4B, and NS5) are located in the C-terminal region of the polyprotein.

Chimeric flaviviruses have been made that include structural and non-structural proteins from different flaviviruses. For example, the so-called ChimeriVax™ technology employs the yellow fever 17D virus capsid and nonstructural proteins to deliver the envelope proteins (prM and E) of other flaviviruses (see, e.g., Chambers et al., J. Virol. 73:3095-3101, 1999). This technology has been used to develop vaccine candidates against dengue, Japanese encephalitis (JE), West Nile (WN), and St. Louis encephalitis (SLE) viruses (see, e.g., Pugachev et al., in New Generation Vaccines, 3rd ed., Levine et al., eds., Marcel Dekker, New York, Basel, pp. 559-571, 2004; Chambers et al., J. Virol. 73:3095-3101, 1999; Guirakhoo et al., Virology 257:363-372, 1999; Monath et al., Vaccine 17:1869-1882, 1999; Guirakhoo et al., J. Virol. 74:5477-5485, 2000; Arroyo et al., Trends Mol. Med. 7:350-354,2001; Guirakhoo et al., J. Virol. 78:4761-4775, 2004; Guirakhoo et al., J. Virol. 78:9998-10008, 2004; Monath et al., J. Infect. Dis. 188:1213-1230, 2003; Arroyo et al., J. Virol. 78:12497-12507, 2004; and Pugachev et al., Am. J. Trop. Med. Hyg. 71:639-645, 2004).

ChimeriVax™-based vaccines have been shown to have favorable properties with respect to properties such as replication in substrate cells, low neurovirulence in murine models, high attenuation in monkey models, high genetic and phenotypic stability in vitro and in vivo, inefficient replication in mosquitoes (which is important to prevent uncontrolled spread in nature), and the induction of robust protective immunity in mice, monkeys, and humans following administration of a single dose, without serious post-immunization side effects. Indeed, the ChimeriVax™-JE vaccine virus, containing the prM-E genes from the SA14-14-2 JE virus (live attenuated JE vaccine used in China), was successfully tested in preclinical and Phase I and II clinical trials (Monath et al., Vaccine 20:1004-1018, 2002; Monath et al., J. Infect. Dis. 188:1213-1230, 2003). Similarly, successful Phase I clinical trials have been conducted with a ChimeriVax™-WN vaccine candidate, which contains prM-E sequences from a West Nile virus (NY99 strain), with three specific amino acid changes incorporated into the E protein to increase attenuation (Arroyo et al., J. Virol. 78:12497-12507,2004).

Other approaches to attenuation, such as mutagenesis of flaviviruses, including chimeric flaviviruses, have been undertaken. These approaches include, for example, the introduction of substitutions in the envelope protein, deletions within the 3'-untranslated region, and deletions in the capsid protein. (See the following references for examples of such mutations: Men et al., J. Virol. 70:3930-3937, 1996; Mandl et al., J. Virol. 72:2132-2140, 1998; Durbin et al., AJTMH 65:405-413, 2001; Pletnev, Virology 282:288-300, 2001; Markoff et al., J. Virol. 76:3318-3328,2002; Kofler et al., J. Virol. 76:3534-3543, 2002; Whitehead et al., J. Virol. 77:1653-1657, 2003; Pletnev et al., Virology 314:190-195, 2003; Pugachev et al., Int. J. Parasitol. 33:567-582, 2003; Bredenbeek et al., J. Gen. Virol. 84:1261-1268,2003; U.S. Patent No. 6,184,024 B1; WO 02/095075; WO 03/059384; WO 03/092592; WO 03/103571; WO 2004/045529; and WO 2006/044857). In another approach, the envelope protein E of ChimeriVax™-JE was probed for permissive insertion sites using a transposon. According to this approach, an inserted transposon in a viable mutant virus is replaced with a desired foreign peptide (see, e.g., WO 02/102828).

Mason and co-workers recently published a new approach to the construction of flavivirus vaccines (RepliVax) based on pseudo-infectious viral particles (PIV) (Mason et al., Virology 351:432-443, 2006). In flavivirus PIVs (thus far described for YF 17D and WN viruses), the capsid protein gene is deleted, with the exception of the 5' cyclization signal sequence occupying ∼ 20 N-terminal codons of C. PIVs are propagated in cells in which the C protein is supplied *in trans.* The latter is necessary for PIV packaging into progeny viral (PIV) particles. Packaged PIVs in the cell culture supernatants are harvested and used as a single-round replication vaccine that induces a potent antibody response, due to the secretion of empty viral particles, as well as an almost complete arsenal of T-cell responses. The robustness of this approach is in part due to the ability of flaviviruses (e.g., YF 17D), and thus PIVs, to infect dendritic cells and activate multiple TLR pathways, enhancing the immune response (Palmer et al., J. Gen. Virol. 88:148-156, 2007; Querec et al., J.E.M. 203:413-424,2006).

In addition to being used as vaccines against flavivirus infection, flaviviruses, such as chimeric flaviviruses, have been proposed for use as vectors for the delivery of other, non-flavivirus antigens. In one example of such a use, a rational approach for insertion of foreign peptides into the envelope protein E of YF17D virus was described, based on knowledge of the tertiary structure of the flavivirus particle, as resolved by cryoelectron microscopy and fitting the known X-ray structure of the E protein dimer into the electron density map (Rey et al., Nature 375:291-298, 1995; Kuhn et al., Cell 108:717-725, 2002). The three-dimensional structure of the E protein trimer in its post-fusion conformation has also been resolved (Modis et al., Nature 427:313-319, 2004; Bressanelli et al., EMBO J. 23:728-738, 2004). Galler and co-workers examined the 3D structures of the E protein dimer and trimer and concluded that the *fg* loop of dimerization domain II should be solvent-exposed in both the dimer and trimer conformations. They used this loop to insert malaria humoral and T-cell epitopes into the E protein of YF17D virus and recovered a few viable mutants (Bonaldo et al., J. Virol. 79:8602-8613, 2005; Bonaldo et al., J. Mol. Biol. 315:873-885,2002; WO 02/072835). Use of this approach, however, does not ensure that a selected site is permissive/optimal for the insertion of every desired foreign peptide in terms of efficient virus replication (as evidenced by some of the Galler et al. data), immunogenicity, and stability. Further, this approach is not applicable to viral proteins for which the 3D structures are unknown (e.g., prM/M, NS1, and most other NS proteins of flaviviruses).

In other approaches, foreign immunogenic proteins/peptides can be expressed within flavivirus vectors if inserted intergenically in the viral ORF. For example, Andino and co-workers attempted to express a model 8-amino-acid anti-tumor CTL epitope flanked by viral NS2B/NS3 protease cleavage sites in several locations within the YF 17D virus polyprotein, e.g., the NS2B/NS1 junction (McAllister et al., J. Virol. 74:9197-9205, 2000). Others have used the NS2B/NS1 site to express an immunodominant T-cell epitope of influenza virus (Barba-Spaeth et al., J. Exp. Med. 202:1179-1184, 2005). Tao et al. expressed a 10-amino acid CTL epitope of malaria parasite at the NS2B-NS3 junction in YF 17D virus, and demonstrated good protection of mice from parasite challenge (Tao et al., J. Exp. Med. 201:201-209, 2005). Recently, we expressed M2e peptide of influenza at the E/NS1 junction (U.S. Serial No. 60/900,672), and Bredenbeek et al. also succeeded in expressing Lassa virus glycoprotein precursor at the E/NS1 junction (Bredenbeek et al., Virology 345:299-304, 2006). Other gene junctions can also be used. In other approaches, foreign antigens have been expressed bi-cistronically (e.g., in the 3'UTR). In other approaches, single-round flavivirus replicons have been developed as recombinant vaccine candidates against various pathogens, and immunogenic potential of recombinant replicons has been demonstrated (Jones et al., Virology 331:247-259, 2005; Molenkamp et al., J. Virol. 77:1644-1648, 2003; Westaway et al., Adv. Virus. Res. 59:99-140, 2003; Herd et al., Virology 319:237-248, 2004; Harvey et al., J. Virol. 77:7796-7803, 2003; Anraku et al., J. Virol. 76:3791-3799, 2002; Varnavski et al., J. Virol. 74:4394-4403, 2000). In a replicon, the prM and E envelope protein genes or the C-prM-E genes are deleted. Therefore, it can replicate inside cells but cannot generate virus progeny (hence single-round replication). It can be packaged into viral particles when the prM-E or C-prM-E genes are provided in trans. Foreign antigens of interest are appropriately inserted in place of the deletion. As in the case of RepliVax, following vaccination, a single round of replication follows, without further spread to surrounding cell/tissues, resulting in immune response against expressed heterologous antigen. Alternatively, immunization can be achieved by inoculation of replicon in the form of naked DNA or RNA. In other approaches, foreign immunogens can be expressed in RepliVax PIVs, e.g., in place of the deleted C gene (Mason et al., Virology 351:432-443, 2006).

Background on influenza. Influenza immunogens were used in this application as model antigens. Influenza virus is a major cause of acute respiratory disease worldwide. Yearly outbreaks are responsible for more than 100,000 hospitalizations and 20,000 to 40,000 deaths in the U.S. alone (Brammer et al., MMWR Surveill. Summ. 51:1-10,2002: Lui et al., Am. J. Public Health 77:712-6, 1987; Simonsen, Vaccine 17:S3-10, 1999; Thompson et al., JAMA 289:179-186, 2003). Approximately 20% of children and 5% of adults worldwide become ill due to influenza annually (Nicholson et al., Lancet 362:1733-1745, 2003). Historically, three subtypes of influenza A virus circulate in human populations, H1N1, H2N2, and H3N2. Since 1968, H1N1 and H3N2 have circulated almost exclusively (Hilleman, Vaccine 20:3068-3087, 2002; Nicholson et al., Lancet 362:1733-1745, 2003; Palese et al., J. Clin. Invest. 110:9-13,2002). Influenza B virus, of which there is only one recognized subtype, also circulates in humans, but generally causes a milder disease than do influenza A viruses. Current inactivated vaccines contain three components, based on selected H1N1 and H3N2 influenza A strains and one influenza B strain (Palese et al., J. Clin. Invest. 110:9-13, 2002). Periodic pandemics, such as the H1N1 pandemic of 1918, can kill millions of people. Influenza experts agree that another influenza pandemic is inevitable and may be imminent (Webby and Webster, Science 302:1519-1522, 2003). The current outbreak of H5N1 avian influenza - the largest on record, caused by a highly lethal strain to humans - has the potential (through mutation and/or genetic reassortment) to become a pandemic strain, with devastating consequences. Another alarming situation arose in 2003 in the Netherlands, where a small but highly pathogenic H7N7 avian influenza outbreak occurred in poultry industry workers. Other subtypes that pose a pandemic threat are H9 and H6 viruses. Although less virulent than the H5 and H7 viruses, both have spread from aquatic birds to poultry during the past 10 years. Further, H9N2 viruses have been detected in pigs and humans (Webby and Webster, Science 302:1519-1522, 2003). Despite the large amount of attention received by avian viruses in the past few years, still the traditional H1, H2, and H3 subtype viruses continue to represent a concern, because highly virulent strains can emerge due to introduction of new antigenically distant strains. For example, H2 viruses are in the high-risk category, because they were the causative agents of the 1957 "Asian" flu pandemic and continue to circulate in wild and domestic ducks.

The current strategy for prevention and control of influenza disease is yearly vaccination against the virus strains likely to be circulating that year. Most licensed influenza vaccines are produced in embryonated chicken eggs and consist of inactivated whole virions or partially purified virus subunits ("split" vaccines). These vaccines are 70 to 90% efficacious in normal healthy adults (Beyer et al., Vaccine 20:1340-1353, 2002). However, efficacy against disease is poorer in the elderly. Live, attenuated intranasal vaccines, also manufactured in embryonated eggs, are available in the U.S. and the former Soviet Union (Treanor et al., In: New Generation Vaccines, 3rd edition. Edited by Levine, M.M. New York, Basel: Marcel Dekker; pp. 537-557, 2004). The U.S. vaccine (Flumist^{®}) is not approved for use in children under 5 or for persons over 55 years of age, the principal target populations for influenza vaccination. Because the major influenza hemagglutinin and neuraminidase proteins recognized by the immune system are continually changing by mutation and reassortment, the vaccine composition has to be altered annually to reflect the antigenic characteristics of the then circulating virus strains. Thus, current vaccines must be prepared each year, just before influenza season, and cannot be stockpiled for use in the case of a pandemic. Moreover, the use of embryonated eggs for manufacture is very inefficient. Only 1 to 2 human doses of inactivated vaccine are produced from each egg. A sufficient supply of pathogen-free eggs is a current manufacturing limitation for conventional vaccines. Even during interpandemic periods, 6 months are typically required to produce sufficient quantities of annual influenza vaccines (Gerdil, Vaccine 21:1776-1779, 2003). There are several development efforts underway to manufacture influenza vaccines in cell culture. However, there are also a number of challenges associated with this approach, in particular the use of unapproved cell lines. Whether eggs or cell cultures are used for vaccine production, reverse genetics or genetic reassortment methods must be employed to convert the new circulating virus strain for which a vaccine is desired into a production strain that replicates to sufficient titer for manufacturing. All of these attributes associated with conventional influenza vaccines are unacceptable in the face of an influenza pandemic.

The development of novel influenza vaccines based on recombinant hemagglutinin (HA) or HA delivered by adenovirus or alphavirus vectors improves manufacturing efficiency, but does not address the problem of annual genetic drift and the requirement to re-construct the vaccine each year.

In summary, the following challenges with current influenza vaccines are recognized:
1. Low efficacy in the case of poor vaccine and virus strain match; limited age range for live cold-adapted vaccines.
2. Requirement to make new vaccines annually to address antigenic changes in the virus.
3. Low manufacturing vaccine yields.
4. Time for construction of appropriate reassortant viruses for manufacture.
5. Insufficient manufacturing capacity to meet the demands of a pandemic.
6. Biosafety concerns during large-scale manufacture of inactivated pathogenic viruses.
7. Adverse reactions in vaccinees allergic to egg products, or due to insufficient attenuation in the case of some live cold-adapted virus vaccines (Treanor et al., In: New Generation Vaccines, 3rd edition. Edited by Levine, M.M. New York, Basel: Marcel Dekker; pp. 537-557,2004).

The 'holy grail' for influenza vaccinology would be a single product that elicits broad, long-lasting protective immunity against all influenza strains, and can be manufactured at high yield and low cost, and stockpiled.

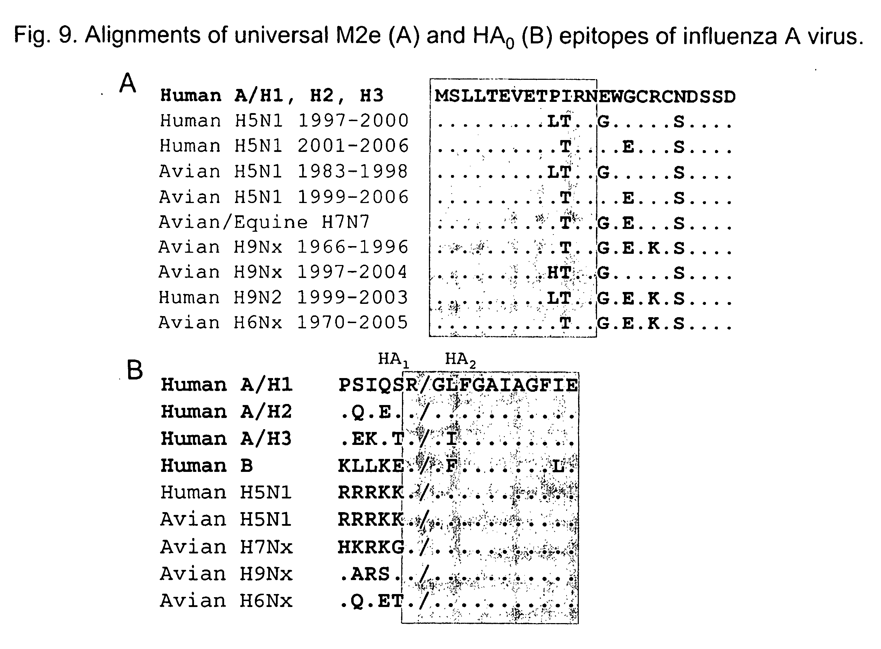

All effective conventional influenza vaccines elicit virus-neutralizing antibodies against HA, which currently represents the immune correlate of protection. However, the antigenicity of HA changes annually. In recent years, other influenza virus proteins have attracted attention as vaccine targets. The M2 protein, and in particular, the ectodomain of M2 (M2e), is highly conserved among influenza A viruses. Shown in Fig. 9A is our alignment of earlier and the most recent human and avian M2e sequences (from http://www.ncbi.nlm.nih.gov/genomes/FLU/FLU/html). Not only is the M2e domain of human influenza viruses conserved among themselves, avian virus M2e sequences are also closely aligned. The highest level of sequence conservation resides in the N-terminal portion of M2e. It is thus extremely noteworthy that it has been shown that the N-terminal 13 amino acids of the M2e peptide (shadowed in the alignment) are primarily responsible for the induction of protective antibodies (Liu et al., FEMS Immunol. Med. Microbiol. 35:141-146, 2003; Liu et al., Immunol. Lett. 93:131-136, 2004; Liu et al., Microbes. Infect. 7:171-177, 2005). This has given rise to the concept of, and hope for, a universal influenza A virus vaccine.

M2e represents the external 23-amino acid portion of M2, a minor surface protein of the virus. While not prominent in influenza virions, M2 is abundantly expressed on the surface of virus-infected cells. However, during normal influenza virus infection, or upon immunization with conventional vaccines, there is very little antibody response to M2 or the M2e determinant. Nevertheless, a non-virus neutralizing monoclonal antibody directed against the M2 protein was shown to be protective in a lethal mouse model of influenza upon passive transfer (Fan et al., Vaccine 22:2993-3003, 2004; Mozdzanowska et al., Vaccine 21:2616-2626, 2003; Treanor et al., J. Virol. 64:1375-1377, 1990). Based on these results, there is considerable interest in M2 and its highly conserved M2e domain as an influenza A vaccine component by a number of vaccine developers.

Antibodies to M2 or M2e do not neutralize the virus but, rather, reduce efficient virus replication sufficiently to protect against symptomatic disease. It is believed that the mechanism of protection elicited by M2 involves NK cell-mediated antibody-dependent cellular cytotoxicity (ADCC). Antibodies against the M2e ectodomain (predominantly of the IgG2a subclass) recognize the epitope displayed on virus-infected cells, which predestines the elimination of infected cells by NK cells (Jegerlehner et al., J. Immunol. 172:5598-1605,2004). Because the immunity elicited by M2 is not sterilizing, limited virus replication is allowed following infection, which serves to stimulate a broad-spectrum anti-influenza immune response. Theoretically, this could lead to a longer, stronger immunologic memory and better protection from subsequent encounters with the same virus or heterologous strains (Treanor et al., In: New Generation Vaccines, 3rd edition. Edited by Levine, M.M. New York, Basel: Marcel Dekker; pp. 537-557,2004).

Walter Fiers and coworkers (Ghent University, Belgium) were among the first to demonstrate the potential of M2e-based vaccines. They genetically fused the M2e determinant to the hepatitis B virus core protein, which when expressed in bacteria, resulted in M2e presentation on the surface of hepatitis B virus core particles (HBc) (Fiers et al., Virus Res. 103:173-176, 2004; Neirynck et al., Nat. Med. 5:1157-1163, 1999). These HBc-M2e particles were shown to be immunogenic in mice and ferrets, and protective in an influenza virus challenge model in each species.

Another conserved influenza virus domain is the maturation cleavage site of the HA precursor protein, HA₀. Its high level of conservation (Macken et al., In: Osterhaus, A. D. M. E., Cox, N., and Hampson A. W. eds., Options for the control of influenza IV. Elsevier Science, Amsterdam, The Netherlands, p. 103-106, 2001) is due to two functional constraints. First, the sequence must remain a suitable substrate for host proteases releasing the two mature HA subunits, HA₁ and HA₂. Second, the N-terminus of HA₂ contains the fusion peptide that is crucial for infection (Lamb and Krug, In: Fields Virology. Fourth edition. Edited by Knipe, D.M., Howley, P.M., Griffin, D.E., et al. Philadelphia: Lippincott Williams and Wilkins; pp. 1043-1126, 2001). The fusion peptide is conserved in both influenza A and B viruses. In a recent report, Bianchi and co-workers (Bianchi et al., J. Virol. 79:7380-7388,2005) demonstrated that a conjugated HA₀ cleavage peptide of influenza B virus elicited protective immunity in mice against lethal challenge with antigenically distant influenza B virus lineages. Remarkably, a conjugated A/H3/HA₀ peptide also protected immunized mice from influenza B challenge. The strictly conserved Arg at the -1 position (the last HA₁ residue preceding the cleavage point), and the +3 and +9 Phe residues (the 3^{rd} and 9^{th} residues of HA₂) were critical for binding of monoclonal antibodies. Thus, the conserved C-terminal portion of the peptide appears to be responsible for protection, suggesting the possibility of making a universal type A and B human influenza virus vaccine based on the HA₀ cleavage domain. Our alignment of the human (H1, H2, H3, and B) HA₀ and all available avian influenza HA₀ sequences (http://www.ncbi.nlm.nih.gov/genomes/FLU/FLU/html) resulted in the consensus sequences (region critical for antibody binding and immunogenicity is shadowed) shown in Fig. 9B.

Recently, a comprehensive on-line database (The Immune Epitope Database and Analysis Resources (IEDB)) became available which captures various epitope data (www.immuneepitope.org). This database was comprehensively analyzed and many cross-protective influenza epitopes, which also can be suitable to construct universal vaccines, were identified (Bui et al., Proc. Natl. Acad. Sci. U.S.A. 104:246-251, 2007 and supplemental tables). Both B- and T-cell promising epitopes were identified (including the HA protective epitope of H3N2 influenza we used below). Most B-cell epitopes are conformational and thus are of considerable size. However, it should be possible to identify permissive sites for such longer epitopes, e.g., in secreted proteins of ChimeriVax viruses, by the direct random insertion approach described in this application. Some highly permissive sites that we found for shorter inserts can be permissive for long inserts (e.g., in prM protein of ChimeriVax-JE, see below).

Various M2e subunit vaccine approaches are being pursued, including peptide conjugates and epitope-displaying particles. However, these approaches require powerful adjuvants to boost the immunogenicity of these weak immunogens. This is particularly critical in the Case of M2e (and likely HAo). Because of the proposed mechanism of protection (ADCC), high levels of specific antibodies are required for efficacy. It is thought that normal serum IgG competes with specific (anti-M2e) IgG for the Fc receptors on NK cells, which are the principal mediators of protection. Thus alternative approaches to universal pandemic influenza vaccines need to be explored. The above description of the medical significance of influenza, the need for an improved universal influenza vaccine, and the availability of appropriate epitopes/antigens of influenza virus provide one example of an important pathogen for which a new vaccine can be created using approaches described in this application. Methods described in this application can be equally applicable to the construction of new/improved vaccines against other pathogens, as described below.

### Summary of the Invention

The invention provides methods for generating viral genomes that include one or more nucleic acid molecules encoding one or more heterologous immunogenic peptides, wherein the viral genomes are the genomes of flaviviruses. These methods include the steps of: (i) providing one or more target viral genes (in, e.g., one or more shuttle vectors or in the context of an intact viral genome); (ii) subjecting the target viral gene to mutagenesis to randomly insert restriction sites; and (iii) ligating a nucleic acid molecule encoding a heterologous immunogenic peptide into the restriction site of the target viral gene to generate a mutant gene library. The methods further include the steps of (iv) transfecting cells with the viral genomes of step (iii) to initiate virus replication, followed by (v) selecting viable (efficiently replicating) virus recombinants enabling efficient presentation of the inserted peptide with an antibody against the immunogenic peptide. When carried out in the context of one or more shuttle vectors, the methods further include the step of introducing the target viral gene, which includes the nucleic acid molecule library encoding the heterologous peptide, into the viral genome from which the target viral gene was derived, in place of the corresponding viral gene lacking the insertion.

The methods of the invention also include generating viral vectors from the viral genomes by introduction of the viral genomes into cells (e.g., Vero cells), as well as isolating viral vectors from the cells or the supernatants thereof. In addition, the target viral genes subject to the methods of the invention can be obtained from viruses that have been subject to this method before (or which have insertions introduced by other means), or viruses lacking insertions.

The methods of the invention can also include subjecting two or more shuttle vectors (e.g., 2, 3, 4, or more), including two or more (e.g., 2, 3, 4, or more) target viral genes, to mutagenesis, and introducing two or more (e.g., 2, 3, 4, or more) target viral genes, including nucleic acid molecules encoding one or more heterologous peptides, into the viral genome, in the place of the corresponding viral genes lacking insertions.

The mutagenesis step of the methods of the invention can involve introduction of one or more transprimers into target viral genes by transposon mutagenesis, whether simultaneously or sequentially. Such transprimers can be removed by endonuclease digestion and nucleic acid molecules encoding heterologous peptides can then be introduced into target viral genes by ligation at the sites of restriction endonuclease digestion. Further, the methods of the invention can involve the generation of libraries of mutated target viral genes.

The viral genomes subject to the methods of the invention are the genomes of flaviviruses, such as chimeric flaviviruses, for example, a chimeric flavivirus that includes the capsid and non-structural proteins of a first flavivirus and the pre-membrane and envelope proteins of a second, different flavivirus. In such an example, the first and second flaviviruses can independently be selected from, for example, the group consisting of Japanese encephalitis, Dengue- 1, Dengue-2, Dengue-3, Dengue-4, Yellow fever, Murray Valley encephalitis, St. Louis encephalitis, West Nile, Kunjin, Rocio encephalitis, Ilheus, tick-borne encephalitis, Central European encephalitis, Siberian encephalitis, Russian Spring-Summer encephalitis, Kyasanur Forest Disease, Omsk Hemorrhagic fever, Louping ill, Powassan, Negishi, Absettarov, Hansalova, Apoi, and Hypr viruses. In addition, intact flavivirus genomes can be subject to the present invention (e.g., yellow fever virus genomes, such as YF17D).

The target viral genes that are subject of the methods of the invention can be, for example, selected from the group consisting of genes encoding envelope, capsid, pre-membrane, NS1, NS2A, NS2B, NS3, NS4A, NS4B, and NS5 proteins.

The heterologous peptides introduced into the viral genomes, according to the methods of the invention, can include one or more vaccine epitopes (e.g., a B-cell epitope and/or a T-cell epitope). The epitopes can be derived from an antigen of a viral, bacterial, or parasitic pathogen. For example, the epitopes can be derived from an influenza virus (e.g., a human or avian influenza virus). In the case of influenza virus epitopes, the heterologous peptides can include, for example, influenza M2e peptides or peptides including an influenza hemagglutinin precursor protein cleavage site (HA0). In other examples, the epitopes are derived from tumor-associated antigens, or allergens. Additional examples of sources (e.g., pathogens) from which heterologous peptides may be obtained, as well as examples of such peptides and epitopes, are provided below.

Also disclosed are viral genomes generated by any of the methods described herein, or the complements thereof. Further, the invention includes flaviviruses comprising such viral genomes, pharmaceutical compositions including such flaviviruses and a pharmaceutically acceptable carrier or diluent. Further described are methods of delivering peptides to patients, involving administering to the patients such pharmaceutical compositions. In one example of such methods, the peptide is an antigen and the administration is carried out to induce an immune response to a pathogen or tumor from which the antigen is derived.

The invention also includes a flavivirus comprising a genome in which at least one nucleic acid molecule encoding one or more heterologous immunogenic peptides is inserted within a sequence encoding one or more proteins selected from the group consisting of capsid, pre-membrane, NS1, NS2A, NS2B, NS3, NS4A, NS4B, and NS5 proteins, whether or not produced by the methods described herein. The flaviviruses can be, e.g., yellow fever viruses (e.g., YF17D) or chimeric flaviviruses (e.g., chimeric flaviviruses including the capsid and non-structural proteins of a first flavivirus and the pre-membrane and envelope proteins of a second, different flavivirus). The first and second flaviviruses of the chimeras can independently be selected from the group consisting of Japanese encephalitis, Dengue-1, Dengue-2, Dengue-3, Dengue-4, Yellow fever, Murray Valley encephalitis, St. Louis encephalitis, West Nile, Kunjin, Rocio encephalitis, Ilheus, tick-borne encephalitis, Central European encephalitis, Siberian encephalitis, Russian Spring-Summer encephalitis, Kyasanur Forest Disease, Omsk Hemorrhagic fever, Louping ill, Powassan, Negishi, Absettarov, Hansalova, Apoi, and Hypr viruses.

Further described are nucleic acid molecules corresponding to the genomes of the flavivirus vectors described above and elsewhere herein, or the complements thereof; pharmaceutical compositions including such viral vectors and a pharmaceutically acceptable carrier or diluent; as well as methods of delivering peptides to patients by administration of such compositions. In one example of such methods, the peptide is an antigen and the administration is carried out to induce an immune response to a pathogen or tumor from which the antigen is derived.

In a specific example, the invention includes a flavivirus comprising a genome in which at least one nucleic acid molecule encoding a heterologous immunogenic peptide is inserted between sequences encoding amino acids 236 and 237 of the non-structural protein 1 (NS1). An additional example, which can exist alone or in combination with other insertions (e.g., the NS1 insert), is a flavivirus comprising a genome in which at least one nucleic acid molecule encoding a heterologous immunogenic peptide is inserted in sequences encoding the amino terminal region of the pre-membrane protein of the vector. This insertion can be located at, for example, position -4, -2, or -1 preceding sequences encoding the capsid/pre-membrane cleavage site, or sequences encoding position 26 of the pre-membrane protein (or a combination thereof). Further, the pre-membrane insertions can include, optionally, a proteolytic cleavage site that facilitates removal of the peptide from the pre-membrane protein.

Specific examples of peptides that can be included in the flaviviruses of the invention include influenza (e.g., human or avian) M2e peptide or a peptide including an influenza (e.g., human or influenza) hemagglutinin precursor protein cleavage site (HA0). These can be naturally occurring or consensus sequences. Additional examples are provided below and elsewhere herein. Further, the vectors can include more than one heterologous peptide, e.g., human and avian influenza M2e peptides. In addition, the flaviviruses of the invention can include one or more second site adaptations, as described herein, which may provide improved properties to the vector (e.g., improved growth characteristics).

Also disclosed are nucleic acid molecules corresponding to the genomes of the flavivirus vectors described herein, or the complements thereof. Further, the invention includes pharmaceutical compositions including the flaviviruses. The compositions can, optionally, include one or more pharmaceutically acceptable carriers or diluents. Further, the compositions can optionally include an adjuvant (e.g., an aluminum compound, such as alum). The compositions may also be in lyophilized form.

Also disclosed are methods of delivering peptides to subjects (e.g., patients, such as human patients, or animals, such as domestic animals or livestock), which involve administration of the compositions described herein. In one example, the methods are carried out to induce an immune response to a pathogen or tumor from which the antigen is derived. In other examples, the methods involve administration of a subunit vaccine. In these examples, the flavivirus vector and the subunit vaccine can be co-administered, the flavivirus vector can be administered as a priming dose and the subunit vaccine can be administered as a boosting dose, or the subunit vaccine can be administered as a priming dose and the flavivirus vector can be administered as a boosting dose. The subunit vaccine can include, for example, hepatitis B virus core particles including a fusion of a heterologous peptide (e.g., an influenza M2e peptide or a peptide including an influenza hemagglutinin precursor protein cleavage site (HA0)) to the hepatitis B virus core protein. These peptides can be naturally occurring or consensus sequences, as described herein.

Also described are methods of making vectors as described herein, involving insertion of sequences encoding peptides of interest into sites identified as being permissive to such insertions (using, e.g., the methods described herein). These vectors can be flavivirus vectors (e.g., yellow fever vectors or chimeric flaviviruses as described herein (e.g., ChimeriVax™-JE or ChimeriVax™-WN)). Exemplary sites for insertion include NS1-236 and positions -4, -2, or -1 preceding the capsid/pre-membrane cleavage site, or position 26 of the pre-membrane protein.

Further described are methods of making pharmaceutical compositions by, for example, mixing any of the vectors described herein with pharmaceutically acceptable carriers or diluents, one or more adjuvants, and/or one or more additional active agents (e.g., a subunit vaccine).

Also disclosed is the use of all of the viral vectors, nucleic acid molecules, and peptides described herein in the preparation of medicaments for use in the prophylactic and therapeutic methods described herein.

The invention provides several advantages. For example, live vaccine viruses (e.g., ChimeriVax™, yellow fever virus, or other live vaccine viruses), as used in the invention, provide significant benefits with respect to the delivery of small polypeptide antigen molecules (e.g., influenza M2e or HA0 cleavage site peptides). The advantages of using live vectors, such as flavivirus-based vectors, include (i) expansion of the antigenic mass following vaccine inoculation; (ii) the lack of need for an adjuvant; (iii) the intense stimulation of innate and adaptive immune responses (YF17D, for example, is the most powerful known immunogen); (iv) the possibility of a more favorable antigen presentation due to, e.g., the ability of ChimeriVax™ (YF17D) to infect antigen presenting cells, such as dendritic cells and macrophages; (v) the possibility to obtain a single-shot vaccine providing life long immunity; (vi) the envelopes of ChimeriVax™ vaccine viruses are easily exchangeable, giving a choice of different recombinant vaccines, some of which are more appropriate than the others in different geographic areas (to make dual vaccines including against an endemic flavivirus, or to avoid anti-vector immunity in a population) or for sequential use; (vii) the possibility of modifying complete live flavivirus vectors into packaged, single-round-replication replicons or PIVs described above, in order to eliminate the chance of adverse events or to minimize the effect of anti-vector immunity during sequential use; (viii) the possibility to combine epitopes inserted using the direct random mutagenesis method described herein with other antigens expressed intergenically, or bicistronically, or in place of deletions in replicons or PIVs to obtain a more robust immune response against one pathogen (if epitopes and other expressed antigens belong to the same pathogen) or two or more pathogens (if epitopes and other antigens expressed belong to different pathogens), and (ix) the low cost of manufacture.

Additional advantages provided by the invention relate to the fact that chimeric flaviviruses of the invention are sufficiently attenuated so as to be safe, and yet are able to induce protective immunity to the flaviviruses from which the proteins in the chimeras are derived and, in particular, the peptides inserted into the chimeras.
Additional safety comes from the fact that some of the vectors used in the invention are chimeric, thus eliminating the possibility of reversion to wild type. An additional advantage of the vectors used in the invention is that flaviviruses replicate in the cytoplasm of cells, so that the virus replication strategy does not involve integration of the viral genome into the host cell, providing an important safety measure. In addition, as is discussed further below, a single flavivirus of the invention can be used to deliver multiple epitopes from a single antigen, or epitopes derived from more than one antigen.

An additional advantage is that the direct random insertion method described herein can result in the identification of broadly permissive sites in viral proteins which can be used directly to insert various other epitopes (as exemplified below for a insertion location in NS1), as well as longer inserts. An additional advantage is that some insertion sites found highly permissive in one flavivirus can be equally permissive in other flaviviruses due to the structure/function conservation in proteins of different flaviviruses. An additional advantage is that recombinant flavivirus bearing an epitope can be used as a booster for, e.g., a subunit vaccine, or a synergistic component in a mixed vaccine composed of, e.g., a subunit or killed vaccine component administered together with the recombinant viral component resulting in a significant enhancement of immune response (as exemplified below for A25 virus mixed together with ACAM-Flu-A subunit vaccine). Further, the described random insertion method can be applied to any flavivirus (or defective flavivirus) genome that has been rearranged, e.g., such as in a modified TBE virus in which the structural protein genes were transferred to the 3' end of the genome and expressed after NS5 under the control of an IRES element (Orlinger et al., J Virol. 80:12197-208, 2006).

Other features and advantages of the invention will be apparent from the following detailed description, the drawings, and the claims.

### Brief Description of the Drawings

Fig. 1 is a schematic illustration of the construction of ChimeriVax™-JE-flu viruses by transposon-mediated random insertion of a consensus M2e peptide into viral prM, E, and/or NS1 glycoproteins. The C and NS2A-NS5 genes can also be targeted for insertion of foreign peptides (e.g., T-cell epitopes) using the approach illustrated in this figure.
Fig. 2 is a schematic illustration of the construction of ChimeriVax™-JE-flu plasmid libraries containing a randomly inserted M2e peptide in prM/M, E, and NS1 genes.
Fig. 3 shows the expression of an influenza A virus consensus M2e protective epitope within the NS1 protein of ChimeriVax™-JE virus, as revealed by staining of viral plaques with antibodies. Viral plaques in 35-mm wells were stained on day 4 post-infection with anti-JE polyclonal antibodies (A) or an anti-M2e monoclonal antibody (B). M2e-positive viral plaques in a 100-mm Petri dish (containing several hundred viral plaques) were stained with an anti-M2e monoclonal antibody (C).
Fig. 4 is a table and a photograph showing the results of an analysis of titers of select purified ChimeriVax™-JE-NS1/M2e viral clones (stocks at P2 level after the last purification step) determined by staining with M2e MAb or JE polyclonal antibodies (table on the left; clones with the highest titers are in bold), and an example of staining for one of the clones (photograph on the right). The results demonstrate the purity of the clones and provide an evidence of high genetic stability.
Fig. 5 is a schematic illustration of the exact location in NS1 gene of ChimeriVax™-JE vector virus, and nt and a.a. sequences of the M2e insert identified by sequencing of viral clones A11 - A92, including clone A25 used in the experiments described below. The entire 105-nt insert is highlighted. The M2e peptide with flanking GG residues on both sides (added for flexibility) is boxed. The BstBI restriction site (TTCGAA) is underlined. Due to the action of a transposon, two viral amino acid residues preceding the insert (SV) were duplicated at the end of the insert (double-underlined).

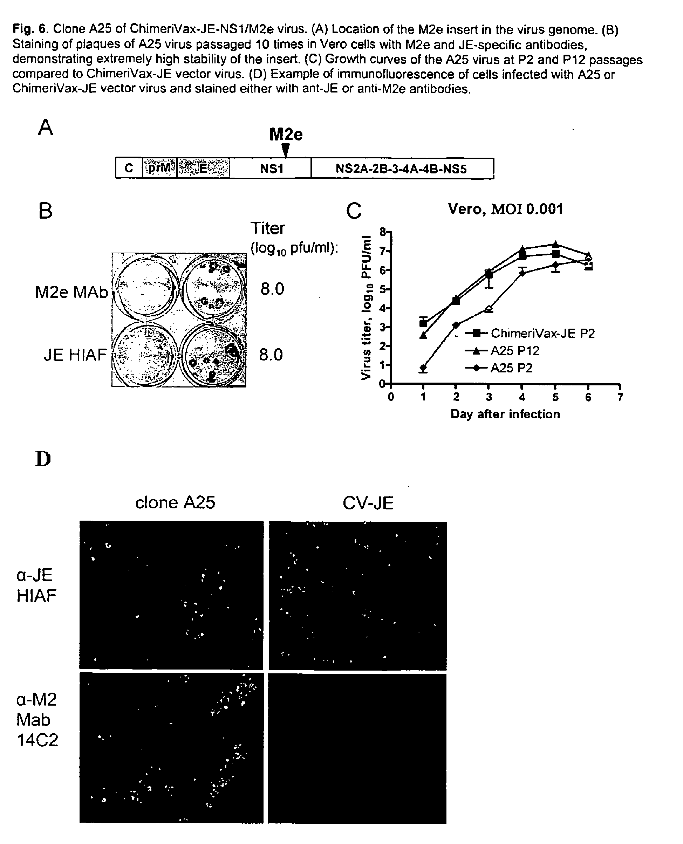

Fig. 6A is a schematic illustration of clone A25 of ChimeriVax™-JE-NS1/M2e virus, which shows the location of the M2e insert in the virus genome. Fig. 6B is a photograph showing the staining of plaques of A25 virus passaged 10 times in Vero cells with M2e and JE-specific antibodies, demonstrating extremely high stability of the insert. Fig. 6C is a graph of growth curves of the A25 virus at P2 and P12 passages as compared to ChimeriVax™-JE vector virus. Panel D is an example of immunofluorescence of cells infected with A25 virus or ChimeriVax-JE vector and stained either with anti-JE or anti-M2e antibodies, also illustrating efficient expression of the M2e epitope by the A25 virus.
Fig. 7 is a graph showing day 54 M2e-specific total IgG: ELISA OD450 values for serially diluted pools of mouse sera from immunized groups 2 and 3 in Table 5.
Fig. 8 is a graph of survival curves for immunized mice shown in Table 5 following IN challenge on day 55 with 20 LD50 of mouse adapted A/PR/8/34 influenza virus.
Fig. 9 is a schematic illustration of alignments of universal M2e (A) and HA₀ (B) epitopes of influenza A virus. The most essential parts of sequences (e.g., for antibody binding) are shadowed.

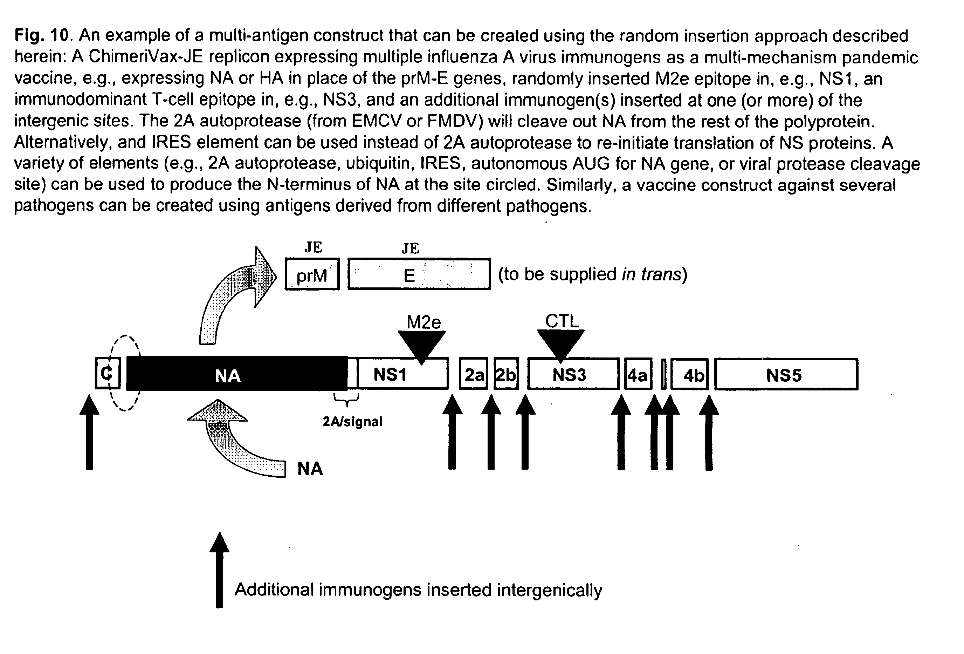

Fig. 10 is an example of a multi-antigen construct that can be created using the random insertion approach described herein: A ChimeriVax-JE replicon expressing multiple influenza A virus immunogens as a multi-mechanism pandemic vaccine, e.g., expressing NA or HA in place of the prM-E genes, randomly inserted M2e epitope in, e.g., NS1, an immunodominant T-cell epitope in, e.g., NS3, and an additional immunogen(s) inserted at one (or more) of the intergenic sites. The 2A autoprotease (from EMCV or FMDV) will cleave out NA from the rest of the polyprotein. Alternatively, an IRES element can be used instead of 2A autoprotease to re-initiate translation of NS proteins. A variety of elements (e.g., 2A autoprotease, ubiquitin, IRES, autonomous AUG for NA gene, or viral protease cleavage site) can be used to produce the N-terminus of NA at the site circled. Similarly, a vaccine construct against several pathogens can be created using antigens derived from different pathogens.
Fig. 11 is an example of M2e antibody-stained Petri dish of Vero cells transfected with ChimeriVax-JE/NS1-M2e RNA library and immediately overlaid with agar, to eliminate competition between viral clones. The RNA for transfection was synthesized on in vitro ligated DNA template obtained by ligation of the NS1-M2e gene library from plasmid pUC-AR03-rM2e into pBSA-AR3-stop vector.
Fig. 12 shows successful expression of M2e peptide in the E protein of ChimeriVax-JE virus: foci of insertion mutants stained with M2e MAb. (A) A variant with the original 35-a.a M2e-containing insert stained on day 6 (experiment 2). (B and C) Variants with 17-a.a. M2e and 17-a.a. M2e flanked with 2 Gly residues, respectively, stained on day 4 (experiment 3).

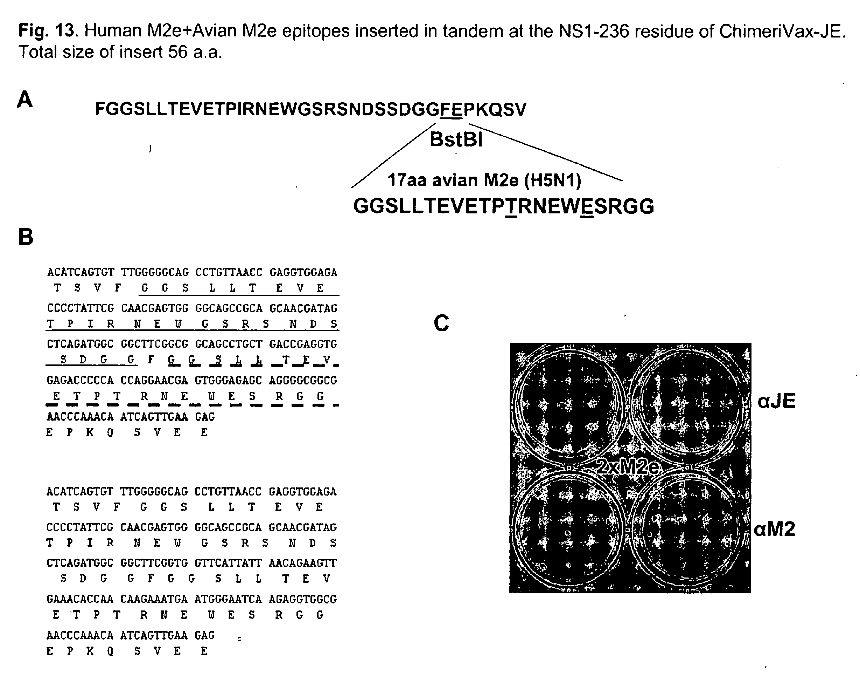

Fig. 13 shows human M2e+Avian M2e epitopes inserted in tandem at the NS1-236 insertion site of ChimeriVax-JE. Total size of insert 56 a.a. (A) Schematic representation of the avian M2e epitope added to the A25 virus. (B) Exact sequences of the two variants of the virus: upper panel shows the sequence of the M2eₕᵤₘₐₙ/M2eₐᵥᵢₐₙ virus constructed using native codons in the avian M2e insert (human M2e is underlined; avian M2e is underlined with dashed line); bottom panel shows the same, except that the avian M2e codons were changed to degenerate codons for higher genetic stability. (C) Plaques of M2eₕᵤₘₐₙ/M2eₐᵥᵢₐₙ virus stained with JE and M2e antibodies.
Fig. 14 shows that ChimeriVax-JE virus tolerates HAtag (influenza H3) B/T-cell epitope at the NS1-236 insertion site identified using the M2e epitope. (A) The insert sequence of the recovered viable virus. (B) Plaques of the virus on Vero cells are stained with anti-HAtag MAb 12CA5.
Fig. 15 shows different modes of foreign epitope expression in flavivirus prM, E, and NS1 proteins.
Fig. 16 shows ChimeriVax-JE insertion variants with M2e in the prM protein. (A) Examples of plaques of M1, M2, M3, M6, and M8 clones, compared to ChimeriVax-JE, determined in one experiment. (B) Growth curves of the prM-M2e clones vs. ChimeriVax-JE vector.
Fig. 17 is a schematic illustration of sequences of ChimeriVax-JE clones with M2e inserts in prM. Most likely and possible signalase cleavage sites predicted by SignalP 3.0 on-line program are shown.
Fig. 18 shows ChimeriVax-WN02 analog of A25 (ChimeriVax-JE/M2eNS1-236) virus: construction and plaques produced on day 6 under agarose overlay and stained with M2e MAb.
Fig. 19 shows ChimeriVax-WN02/A25 and ChimeriVax-WN02/A25adapt viruses. (A) plaques of plaque-purified viral stocks on day 5 produced under methylcellulose overlay, in comparison with the A25 prototype virus and ChimeriVax-WN02. (B) Growth curves in Vero cells, MOI 0.001.

### Detailed Description

Disclosed are methods of generating viral vectors that include heterologous peptides, viral vectors including such peptides, methods of delivering these peptides by administration of the viral vectors in order to, for example, induce an immune response to a pathogen from which an introduced peptide is derived, and compositions including the viral vectors. Details of these viral vectors, peptides, methods, and compositions are provided below.

A central feature of the invention concerns the construction of live, recombinant vaccines by random insertion of immunogenic peptide(s) of a wide range of pathogenic organisms into proteins of live, attenuated vaccine viruses for efficient expression of such peptides in infected cells and presentation to the immune system, with the purpose of inducing strong, long-lasting immunity against target pathogens. For efficient presentation, foreign peptides representing, for example, B-cell epitopes, are randomly inserted into viral proteins, such as proteins that are secreted from infected cells alone (e.g., NS1 and the amino-terminal part of prM of flaviviruses) or in the viral particle (M and E envelope proteins of flaviviruses), in order to stimulate strong anti-peptide antibody responses. Peptides, such as peptides including T-cell epitopes, can be randomly inserted into nonstructural viral proteins, which are synthesized inside infected cells, leading to presentation of the foreign peptides to the immune system via the MHC I/II complex, to induce strong cellular immunity. Insertions into the structural proteins can also lead to efficient MHC-mediated presentation.

As is explained further below, the random fashion of insertion into viral genes according to the present invention allows for selection of the most replication-competent recombinant virus variant(s), providing the highest immunogenicity of the inserted peptide (optimal peptide conformation) and the highest stability of expression. Also as described below, commercially available transposon-mediated insertion systems including, e.g., removable transprimers, can be used as tools for the construction of recombinants of the present invention. The approach of the present invention is described in detail below in the experimental examples section. Briefly, in these examples, a consensus B-cell epitope M2e of the M2 protein of influenza virus (also containing a T-cell epitope), which is highly conserved among type A influenza strains, was inserted into the NS1, prM/M, and E genes of the ChimeriVax™-JE vaccine virus. Multiple virus clones expressing the M2e peptide within the NS1, prM, and E proteins, recognizable by anti-M2e antibodies, were observed, and some were purified and further characterized in vitro/in vivo. In addition, as discussed further below, an NS1 insertion was transferred from the context of ChimeriVax™-JE to ChimeriVax™-WN.

An element of the methods of the invention is the fact that a transposon is used only to randomly insert one or more restriction sites into a desired gene (or genes). Then, a DNA fragment encoding a desired foreign peptide is incorporated into the gene at the restriction site. A mutant gene library can next be incorporated into a complete viral genome (cDNA of an RNA virus), followed by transfection of cells and harvesting heterogeneous viral progeny. The virus "chooses" for itself which insertion locations are more appropriate, not interfering with its viability and efficient replication. A sufficiently high number of mutant virus clones are quickly selected and then tested for high antigenicity using antibodies specific for the inserted peptide, high immunogenicity (proper peptide conformation and presentation to immune cells) by immunizing animals and measuring anti-peptide immune responses and/or protection from challenge, and genetic stability, e.g., by monitoring the presence and expression of the peptide during multiple passages of mutant virus in vitro or in vivo, and genome sequencing to reveal any adaptations that can be valuable for a recombinant vaccine virus biological phenotype (e.g., higher yield during manufacture, higher genetic stability, and higher immunogenicity). As a result, the "best" vaccine virus variant is identified. This "let-the virus-decide" approach thus provides substantial benefits.

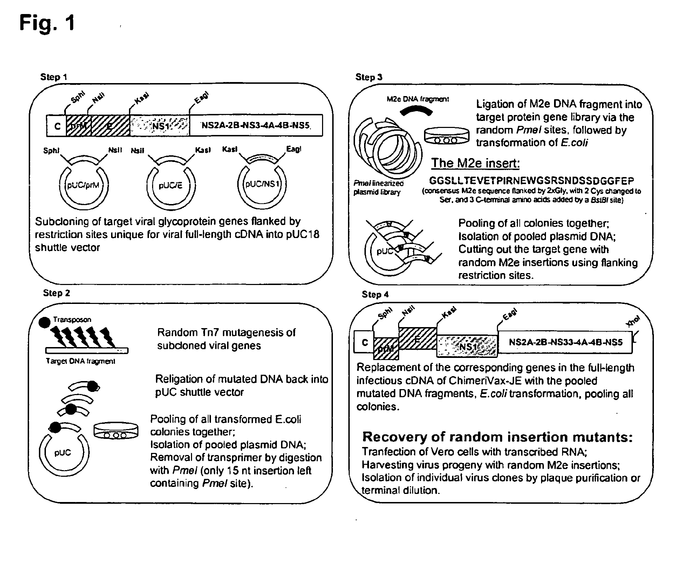

The principle of the random insertion method, which provides a basis for the present invention, is illustrated in Fig. 1. In this example, the M2e peptide of influenza A was introduced into the structural prM/M and E proteins and the nonstructural NS1 protein. The structural proteins are released from the cell as part of viral particle (the N-terminal part of prM may be also secreted), NS1 is transported to the surface of infected cells, and a fraction of it detaches and circulates extracellularly. Extracellular presentation is a prerequisite for strong antibody response. Using NS1 protein for presenting M2e is thus particularly interesting, because the peptide is delivered to the cell surface, mimicking the natural situation with M2 of influenza virus, which may be important for some aspects of M2-mediated immunity; while prM and E protein presentation may lead to a higher immune response, since multiple copies of the peptide will be presented on the surface of viral particles that are presumed to be stronger immunogens.

A restriction site (e.g., a PmeI site) is first randomly incorporated into subcloned target genes (predominantly one site per each gene molecule, although this frequency can be altered, as desired, e.g., by additional rounds of mutagenesis) using, for example, a commercially available kit, such as a New England Biolabs (Beverly, MA) GPS-LS Tn7-mediated mutagenesis kit. The transprimer portion of the transposon is then removed by restriction endonuclease (e.g., PmeI) digestion, and is replaced with an M2e DNA insert, resulting in the generation of a mutant gene plasmid library. The pool of mutated gene molecules is ligated into the full-length cDNA of ChimeriVax™-JE. The DNA template is transcribed in vitro, followed by transfection of cells with the RNA transcripts. Individual clones of viable progeny virus are isolated and tested for the presence of the M2e peptide, immunogenicity, and genetic stability. Further details of this example are described in the experimental examples section, below.

In a variation of the methods described above, mutagenesis takes place in the context of an entire, intact viral genome (e.g., a full-length cDNA of an RNA-containing virus cloned in a plasmid, or complete genomic molecule of a DNA virus), or a DNA fragment encompassing several viral genes, which is followed by recovery of viable insertion mutants. In such an example, the virus not only "chooses" the most appropriate location(s) for the insertion of foreign peptides within a specific target protein, it also chooses the most appropriate target protein encoded within the entire genome or a large fragment of the genome. In another variation, appropriate genes of other vector organisms, such as bacteria (e.g., salmonella, etc.) can be similarly subjected to random insertion mutagenesis followed by selection of that organism's recombinant variants that can be used as vaccines.

In another variation of the methods described herein, more than one transposon is used, either sequentially or simultaneously, to mutagenize the same target gene, in order to randomly insert more than one different immunogenic peptide, followed by selection of viable viral clones carrying different foreign antigenic determinants of one pathogen (for example, to increase immunogenicity/protectiveness), or several pathogens (for example, to create combinatorial vaccine). The random insertion method can also be combined in one virus/vector organism with other expression platforms, e.g., described above (e.g., McAllister et al., J. Virol. 74:9197-205, 2000; Bredenbeek et al., Virology 345:299-304,2006) to generate vaccine candidates expressing several antigens of one pathogen or of different pathogens. Also, additionally expressed proteins can be immunostimulatory molecules, e.g., various known cytokines stimulating appropriate branches of the immune response resulting in increased immunogenicity/efficacy of a recombinant vaccine. In addition, the method can be used to identify broadly permissive insertion sites (e.g., NS1-236 and the N-terminal region ofprM (e.g., amino acids 1-5)). Further, selected promising recombinants can be used as vaccines per se, or in combination with other (e.g., subunit or killed, or other live) vaccines as primers or boosters (if different components are applied sequentially), or as synergistic vaccine components (if different components are inoculated simultaneously).

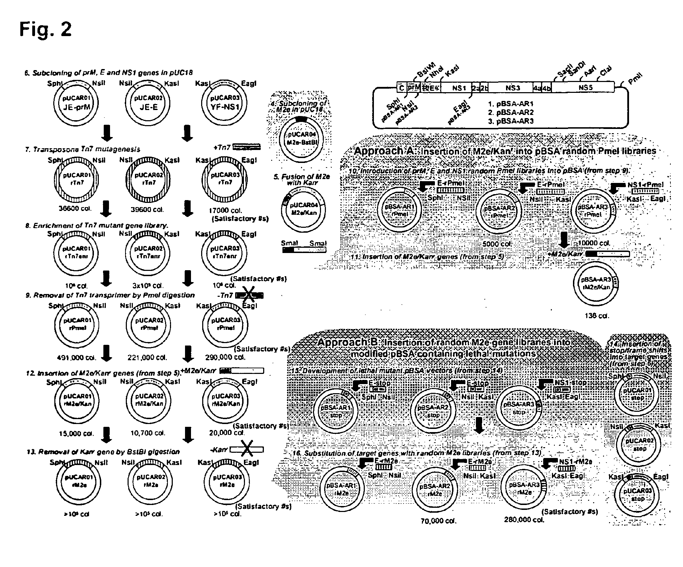

Features of the methods described herein to note include the following: (i) the use of cleavable antibiotic resistance gene (together with epitope insert) to facilitate the generation of plasmid libraries (Fig. 2); (ii) introduction of a stop codon or frameshift into the target gene of full-length plasmid clone (viral cDNA) to minimize the chances of appearance of insert-less virus (Fig. 2); (iii) treatment of plasmid libraries containing random insertions with PmeI enzyme to eliminate any insert-less DNA templates, or doing serial dilutions of transfected cells or RNA used for transfection to minimize competition of insertion mutants with insert-less virus (E-protein expression section); and (iv) easy isolation of insert-containing viral clones using plaque-purification combined with immunostaining of cell monolayers.

### Viral Vectors

Chimeric viruses that can be used in the invention can be based on ChimeriVax™ viruses, which, as described above, consist of a first flavivirus (i.e., a backbone flavivirus) in which a structural protein (or proteins) has been replaced with a corresponding structural protein (or proteins) of a second virus. For example, the chimeras can consist of a first flavivirus in which the prM and E proteins have been replaced with the prM and E proteins of a second flavivirus.

The chimeric viruses that are used in the invention can be made from any combination of viruses. Examples of particular flaviviruses that can be used in the invention, as first or second viruses, include mosquito-borne flaviviruses, such as Japanese encephalitis, Dengue (serotypes 1-4), Yellow fever, Murray Valley encephalitis, St. Louis encephalitis, West Nile, Kunjin, Rocio encephalitis, and Ilheus viruses; tick-borne flaviviruses, such as Central European encephalitis, Siberian encephalitis, Russian Spring-Summer encephalitis, Kyasanur Forest Disease, Omsk Hemorrhagic fever, Louping ill, Powassan, Negishi, Absettarov, Hansalova, Apoi, and Hypr viruses; as well as viruses from the Hepacivirus genus (e.g., Hepatitis C virus).

A specific example of a type of chimeric virus that can be used in the invention is the human yellow fever virus vaccine strain, YF 17D, in which the prM and E proteins have been replaced with prM and E proteins of another flavivirus, such as Japanese encephalitis virus, West Nile virus, St. Louis encephalitis virus, Murray Valley encephalitis virus, a Dengue virus, or any other flavivirus, such as one of those listed above. For example, the following chimeric flaviviruses, which were deposited with the American Type Culture Collection (ATCC) in Manassas, Virginia, U.S.A. under the terms of the Budapest Treaty and granted a deposit date of January 6, 1998, can be used in the invention: Chimeric Yellow Fever 17D/Japanese Encephalitis SA14-14-2 Virus (YF/JE Al.3; ATCC accession number ATCC VR-2594) and Chimeric Yellow Fever 17D/Dengue Type 2 Virus (YF/DEN-2; ATCC accession number ATCC VR-2593).

Details of making chimeric viruses that can be used in the invention are provided, for example, in U.S. Patent Nos. 6,962,708 and 6,696,281; International applications WO 98/37911 and WO 01/39802; and Chambers et al., J. Virol. 73:3095-3101, 1999. In addition, these chimeric viruses can include attenuating mutations, such as those described above and in references cited herein (also see, e.g., WO 2003/103571; WO 2005/082020; WO 2004/045529; WO 2006/044857; WO 2006/116182). Sequence information for viruses that can be used to make the viruses of the present invention is provided, for example, in U.S. Patent No. 6,962,708 (also see, e.g., Genbank Accession Numbers NP_041726; CAA27332; AAK11279; P17763; note: these sequences are exemplary only; numerous other flavivirus sequences are known in the art and can be used in the invention). Additional examples include Genbank accession number NC_002031, which is provided herein as Sequence Appendix 3 (YF17D), Genbank accession number AF315119, which is provided herein as Sequence Appendix 4 (JE-SA-14-14-2), and Genbank accession number AF196835, which is provided herein as Sequence Appendix 5 (West Nile virus). This sequence information is exemplary only, and there are many other flavivirus sequences that can be used in the present invention. Further, these sequences can include mutations as described herein (and in the cited references), be comprised within chimeras as described herein (and in the cited references), and/or include inserts as described herein.

Among the advantages of using the ChimeriVax™ vaccines as vectors in this approach, a main advantage is that the envelope proteins (which are the main antigenic determinants of immunity against flaviviruses, and in this case, anti-vector immunity) can be easily exchanged allowing for the construction of several different vaccines using the same YF17D backbone that can be applied sequentially to the same individual. In addition, different recombinant ChimeriVax™ insertion vaccines can be determined to be more appropriate for use in specific geographical regions in which different flaviviruses are endemic, as dual vaccines against an endemic flavivirus and another targeted pathogen. For example, ChimeriVax™-JE-influenza vaccine may be more appropriate in Asia, where JE is endemic, to protect from both JE and influenza, YF17D-influenza vaccine may be more appropriate in Africa and South America, where YF is endemic, ChimeriVax™-WN-influenza may be more appropriate for the U.S. and parts of Europe and the Middle East, in which WN virus is endemic, and ChimeriVax™-Dengue-influenza may be more appropriate throughout the tropics where dengue viruses are present.

In addition to chimeric flaviviruses, other flaviviruses, such as non-chimeric flaviviruses, can be used as vectors according to the present invention. Examples of such viruses that can be used in the invention include live, attenuated vaccines, such as YF17D and those derived from the YF17D strain, which was originally obtained by attenuation of the wild-type Asibi strain (Smithburn et al., "Yellow Fever Vaccination," World Health Organization, p. 238, 1956; Freestone, in Plotkin et al. (eds.), Vaccines, 2nd edition, W.B. Saunders, Philadelphia, U.S.A., 1995). An example of a YF17D strain from which viruses that can be used in the invention can be derived is YF17D-204 (YF-VAX®, Sanofi-Pasteur, Swiftwater, PA, USA; Stamaril®, Sanofi-Pasteur, Marcy-L'Etoile, France; ARILVAX™, Chiron, Speke, Liverpool, UK; FLAVIMUN®, Berna Biotech, Bern, Switzerland; YF17D-204 France (X15067, X15062); YF17D-204, 234 US (Rice et al., Science 229:726-733, 1985)), while other examples of such strains that can be used are the closely related YF17DD strain (GenBank Accession No. U 17066), YF17D-213 (GenBank Accession No. U17067), and yellow fever virus 17DD strains described by Galler et al., Vaccines 16(9/10):1024-1028, 1998. In addition to these strains, any other yellow fever virus vaccine strains found to be acceptably attenuated in humans, such as human patients, can be used in the invention.

In addition to chimeric flaviviruses and intact flaviviruses, such as yellow fever viruses (e.g., YF 17D vaccine), the methods of the invention can also be used with other, non-flavivirus, live-attenuated vaccine viruses (both RNA and DNA-containing viruses). Examples of such vaccine viruses include those for measles, rubella, Venezuelan equine encephalomyelitis (VEE), mononegaviruses (rhabdoviruses, parainfluenza viruses, etc.), and attenuated strains of DNA viruses (e.g., vaccinia virus, the smallpox vaccine, etc.).

Further, in addition to live viruses, as discussed above, packaged replicons expressing foreign peptides in replicon backbone proteins (e.g., NS1 and other NS proteins, as well as C) can be used in the invention. This approach can be used, for example, in cases in which it may be desirable to increase safety or to minimize antivector immunity (neutralizing antibody response against the envelope proteins), in order to use the same vector for making different vaccines that can be applied to the same individual, or to express several antigens in the same replicon construct. An illustration of such construction is given in Fig. 10. Technology for the construction of single-round replicons is well established, and the immunogenic potential of replicons has been demonstrated (Jones et al., Virology 331:247-259, 2005; Molenkamp et al., J. Virol. 77:1644-1648,2003; Westaway et al., Adv. Virus. Res. 59:99-140, 2003). In an example of such a replicon, most of the prM and E envelope protein genes are deleted. Therefore, it can replicate inside cells, but cannot generate virus progeny (hence single-round replication). It can be packaged into viral particles when the prM-E genes are provided in trans. Still, when cells are infected by such packaged replicons (e.g., following vaccination), a single round of replication follows, without further spread to surrounding cell/tissues. Further, randomly inserted immunologic peptides can be combined with other antigens in the context of PIVs (e.g., Mason et al., Virology 351:432-443, 2006) and any other defective virus vaccine constructs, whole vector viruses, rearranged viruses (e.g., Orlinger et al., J. Virol. 80:12197-12208, 2006), and by means of expression of additional antigens intergenically, bicistronically, in place of PIV deletions, etc.

Protective epitopes from different pathogens can be combined in one virus resulting in triple-, quadruple-, etc., vaccines. Also, a ChimeriVax™ variant containing the envelope from a non-endemic flavivirus can be used to avoid the risk of natural antivector immunity in a population that otherwise could limit the effectiveness of vaccination in a certain geographical area (e.g., ChimeriVax™-JE vector may be used in the U.S. where JE is not present).

Further, the invention includes flaviviruses (e.g., yellow fever viruses, such as YF17D, and chimeric flaviviruses, such as those described herein), that include insertions of one or more heterologous peptides, as described herein, in a protein selected from the group consisting of C, prM, E, NS1, NS2A, NS2B, NS3, NS4A, NS4B, and NS5 proteins, whether or not made by the methods described herein. Methods described in the experimental examples herein for insertions into prM, E, and NS1 teach a person experienced in the art of science precisely how to mutagenize the other flavivirus proteins (C and NS2A-NS5), as well as proteins of other vector viruses, bacteria, etc. Because the C and NS2A-NS5 flavivirus proteins are predominantly expressed intracellularly (with the exception of C, which is also a part of the viral particle), these proteins may be most appropriate for inserting T-cell foreign immunologic epitopes; however B-cell epitopes can be inserted as well, as some antibody response is generated in vivo against most, if not all, of intracellular viral proteins.

### Heterologous Peptides

The flaviviruses of the invention can be used to deliver any peptide or protein of prophylactic or therapeutic value. For example, the flaviviruses of the invention can be used in the induction of an immune response (prophylactic or therapeutic) to any protein-based antigen that is inserted into a virus protein, such as envelope, pre-membrane, capsid, and non-structural proteins of a flavivirus.

The flaviviruses of the invention can each include a single epitope. Alternatively, multiple epitopes can be inserted into the vectors, either at a single site (e.g., as a polytope, in which the different epitopes can be separated by a flexible linker, such as a polyglycine stretch of amino acids), at different sites, or in any combination thereof. The different epitopes can be derived from a single species of pathogen, or can be derived from different species and/or different genuses. The vectors can include multiple peptides; for example, multiple copies of peptides as listed herein or combinations of peptides such as those listed herein. As an example, the vectors can include human and avian M2e peptides (and/or consensus sequences thereof).

Antigens that can be used in the invention can be derived from, for example, infectious agents such as viruses, bacteria, and parasites. A specific example of such an infectious agent is influenza viruses, including those that infect humans (e.g., A, B, and C strains), as well as avian influenza viruses. Examples of antigens from influenza viruses include those derived from hemagglutinin (HA; e.g., any one of H1-H16, or subunits thereof) (or HA subunits HA1 and HA2), neuraminidase (NA; e.g., any one of N1-N9), M2, M1, nucleoprotein (NP), and B proteins. For example, peptides including the hemagglutinin precursor protein cleavage site (HA0) (NIPSIQSRGLFGAIAGFIE for A/H1 strains, NVPEKQTRGIFGAIAGFIE FOR A/H3 strains, and PAKLLKERGFFGAIAGFLE for influenza B strains) or M2e (SLLTEVETPIRNEWGCRCNDSSD) can be used. Other examples of peptides that are conserved in influenza can be used in the invention and include: NBe peptide conserved for influenza B (consensus sequence MNNATFNYTNVNPISHIRGS); the extracellular domain of BM2 protein of influenza B (consensus MLEPFQ); and the M2e peptide from the H5N1 avian flu (MSLLTEVETLTRNGWGCRCSDSSD). Further examples of influenza peptides that can be used in the invention, as well as proteins from which such peptides can be derived (e.g., by fragmentation) are described in US 2002/0165176, US 2003/0175290, US 2004/0055024, US 2004/0116664, US 2004/0219170, US 2004/0223976, US 2005/0042229, US 2005/0003349, US 2005/0009008, US 2005/0186621, U.S. Patent No. 4,752,473, U.S. Patent No. 5,374,717, U.S. 6,169,175, U.S. Patent No. 6,720,409, U.S. Patent No. 6,750,325, U.S. Patent No. 6,872,395, WO 93/15763, WO 94/06468, WO 94/17826, WO 96/10631, WO 99/07839, WO 99/58658, WO 02/14478, WO 2003/102165, WO 2004/053091, WO 2005/055957, and the enclosed Sequence Appendices 1 and 2 (and references cited therein). Further, conserved immunologic/protective T and B cell epitopes of influenza can be chosen from the www.immuneepitope.org database, in which many promising cross-protective epitopes have been recently identified (Bui et al., Proc. Natl. Acad. Sci. U.S.A 104:246-251,2007 and supplemental tables), including one HA epitope of H3N2 virus we used as described below. The invention can employ any peptide from the on-line IEDB resource can be used, e.g., influenza virus epitopes including conserved B and T cell epitopes described in Bui et al., supra.

Protective epitopes from other human/veterinary pathogens, such as parasites (e.g., malaria), other pathogenic viruses (e.g., human papilloma virus (HPV), herpes simplex viruses (HSV), human immunodeficiency viruses (HIV; e.g., gag), and hepatitis C viruses (HCV)), and bacteria (e.g., *Mycobacterium tuberculosis, Clostridium difficile,* and *Helicobacter pylori*) can also be included in the vectors of the invention. Various appropriate epitopes of these and other pathogens can be easily found in the literature. For example, cross-protective epitopes/peptides from papilomavirus L2 protein inducing broadly cross-neutralizing antibodies that protect from different HPV genotypes have been identified by Schiller and co-workers, such as amino acids 1-88, or amino acids 1-200, or amino acids 17-36 of L2 protein of, e.g., HPV16 virus (WO 2006/083984 A1; QLYKTCKQAGTCPPDIIPKV). Examples of additional pathogens, as well as antigens and epitopes from these pathogens, which can be used in the invention are provided in WO 2004/053091, WO 03/102165, WO 02/14478, and US 2003/0185854.

Additional examples of pathogens from which antigens can be obtained are listed in Table 1, below, and specific examples of such antigens include those listed in Table 2. In addition, specific examples of epitopes that can be inserted into the vectors of the invention are provided in Table 3. As is noted in Table 3, epitopes that are used in the vectors of the invention can be B cell epitopes (i.e., neutralizing epitopes) or T cell epitopes (i.e., T helper and cytotoxic T cell-specific epitopes).

The vectors of the invention can be used to deliver antigens in addition to pathogen-derived antigens. For example, the vectors can be used to deliver tumor-associated antigens for use in immunotherapeutic methods against cancer. Numerous tumor-associated antigens are known in the art and can be administered according to the invention. Examples of cancers (and corresponding tumor associated antigens) are as follows: melanoma (NY-ESO-1 protein (specifically CTL epitope located at amino acid positions 157-165), CAMEL, MART 1, gp100, tyrosine-related proteins TRP1 and 2, and MUC1); adenocarcinoma (ErbB2 protein); colorectal cancer (17-1A, 791Tgp72, and carcinoembryonic antigen); prostate cancer (PSA1 and PSA3). Heat shock protein (hsp110) can also be used as such an antigen.

In another example of the invention, exogenous proteins that encode an epitope(s) of an allergy-inducing antigen to which an immune response is desired can be used. In addition, the vectors of the invention can include ligands that are used to target the vectors to deliver peptides, such as antigens, to particular cells (e.g., cells that include receptors for the ligands) in subjects to whom the vectors administered.

The size of the peptide or protein that is inserted into the vectors of the invention can range in length from, for example, from 3-1000 amino acids in length, for example, from 5-500, 10-100, 20-55, 25-45, or 35-40 amino acids in length, as can be determined to be appropriate by those of skill in the art. As discussed elsewhere herein, the amino terminal pre-membrane insertions described herein provide the possibility of longer insertions (see below). Further, the peptides noted herein can include additional sequences or can be reduced in length, also as can be determined to be appropriate by those skilled in the art. The peptides listed herein can be present in the flaviviruses of the invention as shown herein, or can be modified by, e.g., substitution or deletion of one or more amino acids (e.g., 1, 2,3,4, 5,6,7, 8,9,10, or more amino acids). In addition, the peptides can be present in the flaviviruses in the context of larger peptides.

The invention also includes the identification and use of broadly permissive insertion sites such as, for example, NS1-236, into which multiple different peptides can be inserted, as shown in the context of two different chimeras (see below). Additional broadly permissive sites include the amino terminal region of prM of chimeric viruses including ChimeriVax™-JE and ChimeriVax™-WN (see below). Insertions may be made in such viruses in any one or more of positions 1-50, e.g., 1-25, 1-15, 1-10, or 1-5.

Further, the invention includes the identification and use of second site adaptations that are obtained by, for example, cell (e.g., Vero) culture. Such adaptations may provide benefits such as increased replication, etc. Specific examples of such adaptations, which can be used in other contexts, are described below in the experimental examples.

### Production and Administration

The viruses described above can be made using standard methods in the art. For example, an RNA molecule corresponding to the genome of a virus can be introduced into primary cells, chicken embryos, or diploid cell lines, from which (or the supernatants of which) progeny virus can then be purified. Other methods that can be used to produce the viruses employ heteroploid cells, such as Vero cells (Yasumura et al., Nihon Rinsho 21:1201-1215, 1963). In an example of such methods, a nucleic acid molecule (e.g., an RNA molecule) corresponding to the genome of a virus is introduced into the heteroploid cells, virus is harvested from the medium in which the cells have been cultured, harvested virus is treated with a nuclease (e.g., an endonuclease that degrades both DNA and RNA, such as Benzonase™; U.S. Patent No. 5,173,418), the nuclease-treated virus is concentrated (e.g., by use of ultrafiltration using a filter having a molecular weight cutoff of, e.g., 500 kDa), and the concentrated virus is formulated for the purposes of vaccination. Details of this method are provided in WO 03/060088 A2. Further, methods for producing chimeric viruses are described in the documents cited above in reference to the construction of chimeric virus constructs.

The flaviviruses of the invention can be administered in amounts and by using methods that can readily be determined by persons of ordinary skill in this art. In the case of chimeric flaviviruses and yellow fever virus-based vectors, the vectors can be administered and formulated, for example, in the same manner as the yellow fever 17D vaccine, e.g., as a clarified suspension of infected chicken embryo tissue, or a fluid harvested from cell cultures infected with the chimeric yellow fever virus. The flaviviruses of the invention can thus be formulated as sterile aqueous solutions containing between 100 and 1,000,000 infectious units (e.g., plaque-forming units or tissue culture infectious doses) in a dose volume of 0.1 to 1.0 ml, to be administered by, for example, intraperitoneal, intramuscular, subcutaneous, or intradermal routes (see, e.g., WO 2004/0120964 for details concerning intradermal vaccination approaches). In addition, because flaviviruses may be capable of infecting the human host via the mucosal routes, such as the oral route (Gresikova et al., "Tick-borne Encephalitis," In The Arboviruses, Ecology and Epidemiology, Monath (ed.), CRC Press, Boca Raton, Florida, 1988, Volume IV, 177-203), the vectors can be administered by a mucosal route.

When used in immunization methods, the vectors can be administered as a primary prophylactic agent in adults or children at risk of infection by a particular pathogen. The vectors can also be used as secondary agents for treating infected patients by stimulating an immune response against the pathogen from which the peptide antigen is derived. For example, a recombinant expressing epitopes from E6/E7 proteins, or whole E6/E7 proteins, of HPV can be used as a therapeutic HPV vaccine.

For vaccine applications, optionally, adjuvants that are known to those skilled in the art can be used. Adjuvants that can be used to enhance the immunogenicity of the chimeric vectors include, for example, liposomal formulations, synthetic adjuvants, such as (e.g., QS21), muramyl dipeptide, monophosphoryl lipid A, or polyphosphazine. Although these adjuvants are typically used to enhance immune responses to inactivated vaccines, they can also be used with live vaccines. In the case of a chimeric vector delivered via a mucosal route, for example, orally, mucosal adjuvants such as the heat-labile toxin of *E. coli* (LT) or mutant derivations of LT can be used as adjuvants. In addition, genes encoding cytokines that have adjuvant activities can be inserted into the vectors. Thus, genes encoding cytokines, such as GM-CSF, IL-2, IL-12, IL-13, or IL-5, can be inserted together with foreign antigen genes to produce a vaccine that results in enhanced immune responses, or to modulate immunity directed more specifically towards cellular, humoral, or mucosal responses. Alternatively, cytokines can be delivered, simultaneously or sequentially, separately from a recombinant vaccine virus by means that are well known (e.g., direct inoculation, naked DNA, in a viral vector, etc.).

The flaviviruses of the invention can be used in combination with other vaccination approaches. For example, the viruses can be administered in combination with subunit vaccines including the same or different antigens. The combination methods of the invention can include co-administration of flaviviruses of the invention with other forms of the antigen (e.g., subunit forms or delivery vehicles including hepatitis core protein (e.g., hepatitis B core particles containing M2e peptide on the surface produced in E. coli (HBc-M2e; Fiers et al., Virus Res. 103:173-176, 2004))). Alternatively, the flaviviruses of the present invention can be used in combination with other approaches (such as subunit or HBc approaches) in a prime-boost strategy, with either the flaviviruses of the invention or the other approaches being used as the prime, followed by use of the other approach as the boost, or the reverse. Further described are prime-boost strategies employing the flaviviruses of the present invention as both prime and boost agents.

In addition to vaccine applications, as those skilled in the art can readily understand, the vectors of the invention can be used in gene therapy methods to introduce therapeutic gene products into a patient's cells and in cancer therapy. Further, recombinant viruses, e.g., chimeric or intact flaviviruses described herein, containing an immunologic epitope can be used in prime/boost regimens to enhance efficacy of subunit or whole-organism killed vaccines, similarly to recombinant alphavirus replicons (US 2005/0208020 A1). Further, some of our results below also demonstrate a strong synergistic effect between a flavivirus containing a foreign epitope (e.g., ChimeriVax-JE/NS1-M2e) and a subunit vaccine (e.g., HBc-M2e) when the two are mixed and inoculated simultaneously. The later may result in new, efficient combined vaccine formulations not requiring adjuvants and providing new desirable features, e.g., Th1 shift in immune response. In addition, foreign epitopes can be expressed on the surface of viral particles (in prM-E) as described herein, however instead of using recombinant virus as a live vaccine, it can be inactivated, e.g., using formalin, and used as a killed vaccine. Such approach can be particularly applicable if vector virus is a wild type virus, which can be pathogenic for humans/animals.

### Experimental Examples

The following experimental examples show the insertion of M2e sequences into ChimeriVax™-JE, as well as an HA epitope. Sequences were also inserted into a ChimeriVax™-WN construct. The methods described in this example can also be used with other viruses, such as other chimeric flaviviruses and virus-based vectors (e.g., replicons and PIVs), as well as other vector organisms, as described above, to insert sequences into other proteins, and to insert other peptides.

The yellow fever 17D (YF17D) live attenuated vaccine strain has been used in humans for the past 60 years, has an excellent safety record, and provides long-lasting immunity after administration of a single dose. As is noted above, ChimeriVax™-JE is a live, attenuated recombinant vaccine strain in which the genes encoding certain structural proteins (PrME) of YF17D have been replaced with the corresponding genes from the genetically attenuated Japanese encephalitis (JE) virus SA14-14-2. Both capsid and all nonstructural (NS) genes responsible for intracellular replication of this chimera are derived from the YF17D vaccine strain. Similarly, ChimeriVax™-WN is a live, attenuated recombinant vaccine strain in which the genes encoding PrM and E proteins of YF17D have been replaced with the corresponding genes from a West Nile virus strain. An example of such a chimera employs the sequence of West Nile virus strain NY99-flamingo 382-99 (GenBank Accession Number AF196835). In a further example, herein referred to as ChimeriVax™-WN02, in the NY99-flamingo 382-99 envelope sequence, lysine at position 107 is replaced with phenylalanine, alanine at position 316 is replaced with valine, and lysine at position 440 is replaced with arginine.

This section describes the plasmid construction steps that are illustrated in Fig. 2. Construction began with a pBSA single-plasmid construct containing the entire cDNA of ChimeriVax™-JE virus, based on a pBeloBac11 low-copy number vector. This plasmid was constructed by assembling the ChimeriVax™-JE-specific cDNA portions (together with an SP6 promoter) of the YFM5'3'SA14-14-2 and YF5.2SA14-14-2 plasmids (the original two plasmids for ChimeriVax™-JE) in one low copy number vector pBeloBac11 (New England Biolabs, Beverly, MA). The plasmid contains several unique restriction sites, which are convenient for gene subcloning (shown above the virus genome in the upper right plasmid diagram in Fig. 2). Additional restriction sites, SphI, NsiI, and EagI, used for subcloning of the prM, E, and NS1 genes, were introduced into the pBSA plasmid by silent site-directed mutagenesis (Steps 1-3 in Fig. 2).

The three target genes were subcloned into a pUC18 plasmid vector (Step 6) and the resulting plasmids were randomly mutated using a Tn7 transposon (Step 7). Transformed *E. coli* were grown in the presence of chloramphenicol (a chloramphenicol resistance gene is encoded by a removable transprimer of the transposon), and three mutated plasmid libraries represented by large numbers of bacterial colonies were prepared. During preparation of the mutant plasmid libraries, the number of colonies in each library was at least 3 times higher than the number of nucleotides in the mutated DNA sequence, to ensure that a foreign insert of interest (encoding a peptide such as M2e) is subsequently incorporated after every nucleotide of target gene. The numbers of colonies in each library are shown in Fig. 2. The mutated prM, E, and NS1 gene libraries were subcloned in a pUC18 vector (Step 8), and the transprimers were removed by PmeI digestion and re-ligation (Step 9), leaving behind only a 15 nucleotide random insert containing a unique PmeI site in each gene molecule. To facilitate insertion of M2e, a SmaI-SmaI cassette containing M2e and a kanamycin resistance gene was first assembled (Steps 4-5). The Kan^{r} gene can be removed from this cassette by digestion at engineered flanking BstBI sites. The cassette was inserted at the PmeI sites in the libraries from Step 9, with selection of new M2e-containing libraries being achieved by growing bacteria in the presence of Kan (Step 12). The native human influenza A M2e consensus sequence, SLLTEVETPIRNEWGCRCNDSSD, used in the construction was modified in that the two Cys residues were changed to Ser to avoid any unwanted S-S bridging, which does not affect the antigenicity/immunogenicity of the peptide, and two Gly residues were added on both sides for flexibility (GGSLLTEVETPIRNEWGSRSNDSSDGG). The Kan^{r} gene was then removed from the resulting gene libraries containing random M2e inserts by digestion with BstBI (Step 13).

In one approach (Approach A in Fig. 2), to produce ChimeriVax™-JE-flu template cDNA libraries with M2e inserted randomly in viral prM, E, and NS1 genes, mutant gene libraries from Step 9 (containing random PmeI sites) are cloned into modified pBSA plasmids from Steps 1-3. However, when we first attempted to insert the M2e/Kan^{r} cassette into the pBSA-AR3-rPmeI library from Step 10, the number of bacterial clones in the resulting pBSA-AR3-rM2e/Kan library, grown in the presence of Kan, was low (Step 11). Notwithstanding, this approach allows rapid construction of libraries containing any immunogenic epitopes (e.g., from malaria parasite, TB, viral pathogens, etc.).

In another approach (Approach B), a stop codon/frameshift modification was first introduced into subcloned prM, E, and NS1 genes (Step 14), and the modified genes, containing mutations lethal for the virus, were introduced into pBSA-AR1-3 plasmids (Step 15). This was done to eliminate the possibility of appearance of nonmutant ChimeriVax™-JE virus following transfection of cells due to the presence of a proportion of contaminating nonmutant template in a final ChimeriVax™-JE-flu template library. The final, full-length template libraries for ChimeriVax™-JE-flu viruses were obtained by replacing the target gene fragments in libraries from Step 15 with those containing random M2e inserts from Step 13 (Step 16).

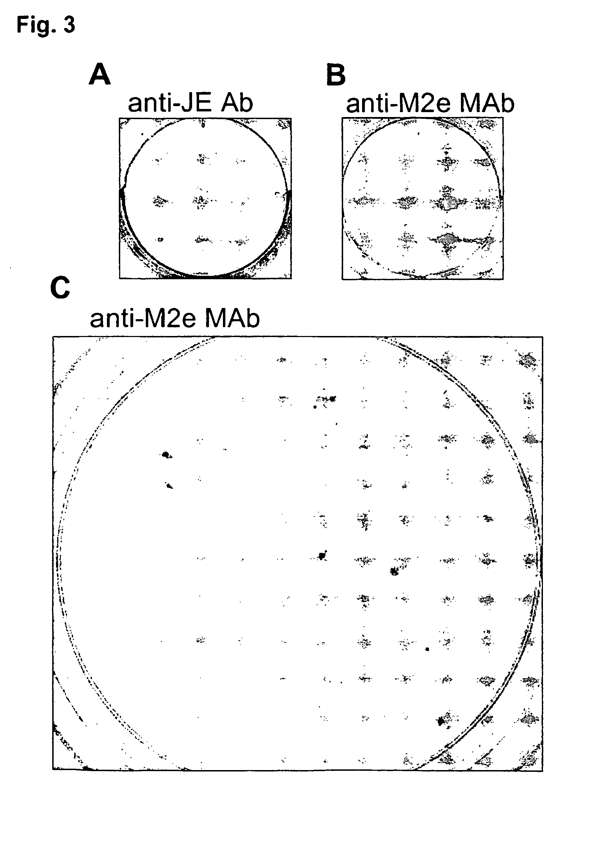

To produce ChimeriVax™-JE-flu viruses with the consensus M2e sequence randomly inserted in NS1, the pBSA-AR3-rM2e plasmid library was linearized with XhoI (an XhoI site is located at the end of viral cDNA) and transcribed in vitro with SP6 RNA polymerase (an SP6 promoter is located upstream from viral cDNA), followed by transfection of Vero cells. Virus progeny was harvested when a cytopathic effect was first detectable or pronounced, on days 3-6 post-transfection. Viral titers in harvested samples were determined by plaque assay (methyl-cellulose overlay) with staining of methanol-fixed monolayers using mouse hyperimmune anti-JE acsitic fluid (ATCC) to detect all plaques, or a commercially available monoclonal antibody (Mab) 14C2 against influenza M2e epitope was used to detect only plaques expressing M2e peptide recognizable by the Mab. Overall titers were in excess of 7 log₁₀ pfu/ml. M2e-positive plaques were readily detectable and represented up to 0.4% of total plaques (Figs. 3A and 3B). Some of these M2e-positive plaques were as large as M2e-negative plaques, indicating efficient virus replication. A majority of the total plaques were M2e negative, which could be because the insert at some of random locations in NS1 is unstable, resulting in appearance of non-mutant ChimeriVax™-JE virus shortly after transfection. Alternatively, the insert may be present but inaccessible to antibodies.

Several techniques can be used to isolate individual positive virus clones. We combined plaque purification with MAb staining (immunofocus assay). In this assay, Vero cells infected with serial dilutions of virus are overlaid with agarose. On day 5, agarose is removed and the cell monolayer (e.g., in a Petri dish; Fig. 3C) is fixed with methanol and stained with a MAb. The agarose is then aligned with the Petri dish and portions of the gel corresponding to positive M2e-plaques are harvested and frozen. Alternatively, cell monolayers were stained by Mab without methanol fixation. Cells in positive plaques were carefully scraped from the plastic and frozen. Roughly 80 candidate virus clones have been isolated using this procedure, and are being further purified by one to two additional rounds of plaque-purification. Another method we have used combined terminal dilution of virus, harvesting cell supernatants, and staining of cell monolayers in 96-well plates with the MAb to identify positive wells at highest possible dilution (ideally infected with a single positive viral particle). This method resulted in 37 candidate clones. Further analysis demonstrated that one of these appears to be a pure clone, while the rest are still mixed with M2e negative virus.

A sufficiently large number of M2e-positive clones (e.g., 50-100) can next be tested for immunogenicity and protective efficacy (including long-term protection) against challenge with wild type influenza virus in mice (and/or ferrets) using available animal models and methods to measure anti-M2e antibody titers in mouse sera (e.g., ELISA using a synthetic M2e peptide to measure total IgG/IgM or isotypic IgG1/IgG2 antibodies) as well as activity in an in vitro ADCC test. Genetic stability can be evaluated by serial passage of viruses in cell culture (or in vivo), followed by immunofocus assay and/or sequencing.

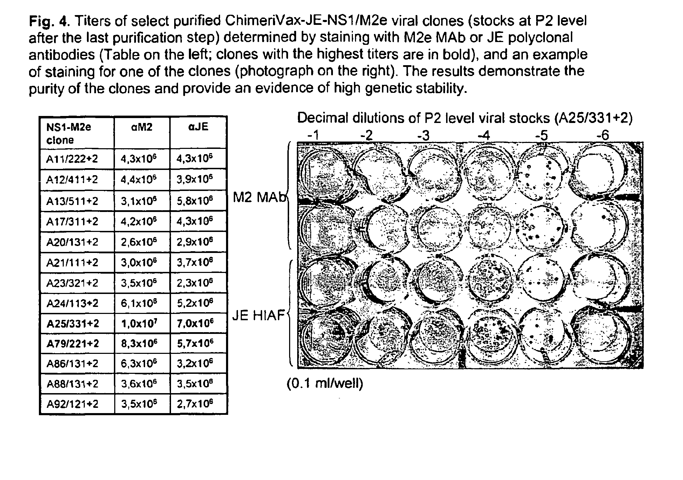

In further developments, following the last of 3-4 plaque-purification steps done starting from virus harvested after transfection, viral stocks of 13 clones were produced by two amplification passages in Vero cells. These amplified samples were designated P2 research viral stocks (passage 2 after purification). Titers of the stocks were determined to be in the range of 2.6x10⁶-1.0x10⁷ pfu/mL. Importantly, staining with both M2e MAb and JE HIAF produced nearly identical titers (Fig. 4), indicating that the viral stocks were pure. In addition, this result was the first evidence of genetic stability of the recombinant viruses. If the viruses were not pure or stable, the non-mutant ChimeriVax™-JE virus would outgrow the M2e-expressing recombinants, which clearly was not the case. In addition, efficient M2e staining of viral plaques was observed both with methanol fixation of cells (detecting intracellular and surface protein) and without methanol fixation (detecting only surface protein). Thus, NS1 protein containing M2e peptide, as expected, was transported normally to the surface of infected cells and most likely also secreted. NS1 therefore enabled efficient surface/extracellular presentation of the epitope, which is highly desirable for the induction of robust anti-M2e antibody response in vivo.

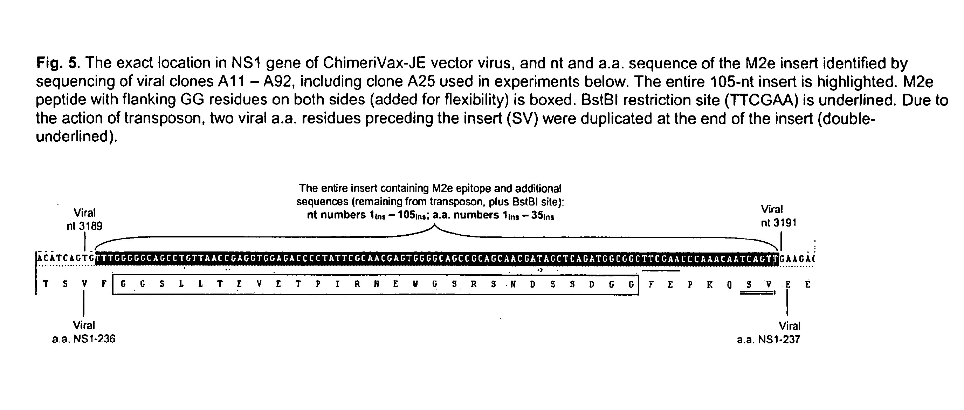

The NS1 gene of the 13 clones (A11 - A92 in Fig. 4) was sequenced to determine the locations of their M2e insert. Surprisingly, the 35-amino acid insert was found to be located at exactly the same site in all 13 clones, in the C-terminal half of the NS1 protein, after nucleotide 3190 of the ChimeriVax™-JE virus genome, between viral NS1 amino acid residues 236 and 237. The exact sequence of the insert and surrounding NS1 nucleotide and amino acid residues are shown in Fig. 5.

The most likely explanation for the insert being present in the same location in all 13 clones is that the clones were plaque-isolated from virus harvested up to 6 days after transfection of Vero cells, when CPE was observed. Competition between different initial variants (having inserts at different locations) has occurred during virus replication prior to harvest, and one variant may have become dominant in the viral population. Therefore, the 13 picked clones represented one insertion variant.

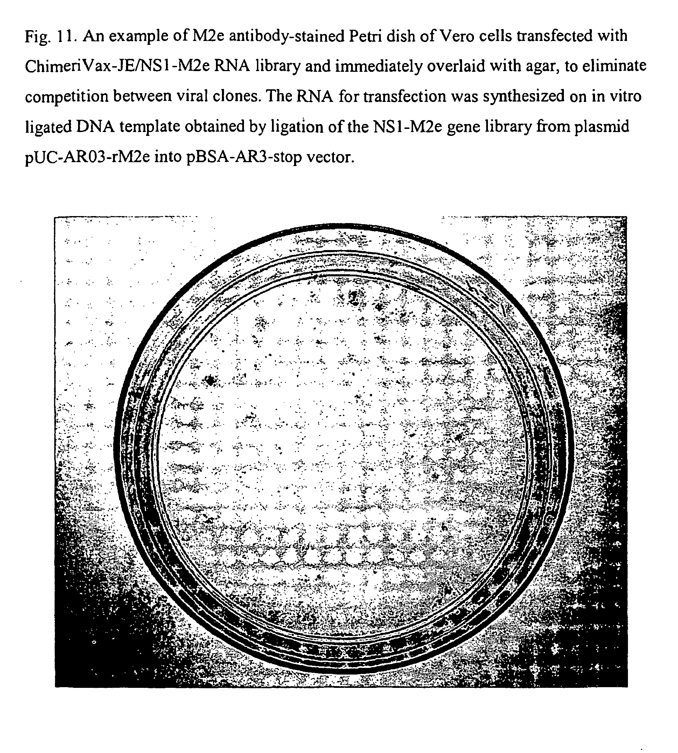

To overcome the problem of competition between variants, additional clones can be prepared by plaque-picking done immediately after transfection (e.g., to find a more immunogenic vaccine candidate, if necessary). In this later approach, Vero cells are transfected with in vitro-synthesized RNA and immediately overlaid with agarose, followed by staining of cells with M2e antibody and harvesting positive clones from the agarose. We have attempted this using RNA transcripts for transfection produced by either transcribing in vitro the pBSA-AR3 plasmid library (Fig. 2), or by in vitro ligation of the NS1-M2e gene library from plasmid pUC-AR03-rM2e (which was found to be more representative than the pBSA-AR3 library) into pBSA-AR3-stop vector (Fig. 2). Agarose overlay was removed on day 4-5, and the cell monolayer was stained with M2e MAb. Multiple positive viral foci of varying sizes were observed. An example of a stained Petri dish of Vero cells transfected with RNA obtained using the in vitro DNA ligation step is shown in Fig. 11. Portions of the agarose corresponding to several larger positive plaques were collected and then further purified by additional rounds of plaque purification. Interestingly, when the new variants were sequenced, they had the same location of M2e insertion as in A25 virus. This identifies NS1-236 as a highly permissive site in the NS1 protein, which yielded highly efficiently replicating insertion mutants, producing the largest plaques. Nevertheless, judged by the variable sizes of foci in Fig. 11, it seems clear that the M2e insert intercalated at different locations within NS1. Some less efficiently replicating variants, forming intermediate or small plaques, may be of practical value.

We have also used the BstBI restriction site located at the end of M2e insert of the A25 clone (Fig. 5) to add a second influenza protective epitope at this NS1 location. For example, we have incorporated the M2e epitope from H5N1 avian influenza flanked with 2xGly linkers for flexibility (as shown schematically in Fig. 13A), and obtained viable virus. Thus, the latter insertion mutant contains a tandem of human influenza M2e followed by avian influenza M2e. This virus could be a universal vaccine capable of protecting the population from both human influenza A strains and avian flu. In this construction, the NS1 gene with human M2e insert from A25 virus was first cloned into the ChimeriVax-JE infectious clone by means of reverse genetics. Avian M2e sequences were then added by cloning at the BstBI site a double-stranded DNA fragment composed of two annealed phosphorylated oligonucleotides. Two versions of M2eₕᵤₘₐₙ/M2eₐᵥᵢₐₙ virus were constructed, one with native M2e sequence of H5N1 influenza (except that the penultimate Cys was changed to Ser; the sequence shown in the upper panel of Fig. 13B), and the other in which native H5N1 codons were replaced with degenerate codons to minimize nucleotide sequence similarity with the upstream human M2e sequence (sequence shown in bottom panel of Fig. 13B). The latter was done in order to reduce the chances of homologous recombination in the recombinant virus, between human M2e and avian M2e sequences, which should result in higher genetic stability of the virus. Plaques of constructed viruses were stained with both JE and M2e specific antibodies (Fig 13C). Plaque size was somewhat reduced compared to the A25 parent virus. Titers of P1 viruses harvested immediately after transfection were reasonably high (∼ 5 log₁₀ pfu/ml). Although the viruses have not been further passaged in Vero cells, higher titers can be expected at P2 and subsequent passages, as titers at P2 are usually higher for ChimeriVax constructs as compared to P1. This experiment clearly demonstrates that longer inserts (in this case 56 amino acids in length together with the few extra-residues from the transposon) can be incorporated at an insertion site identified using a shorter insert (the 35-amino acid M2e epitope in A25 virus). The NS1-236 insertion location tolerates inserts of at least 56 amino acids. Another important conclusion is that the addition of avian M2e sequence to the human M2e sequence changed the overall insert sequence (and possibly structure) at the NS1-236 location in comparison to the A25 virus. This was the first experimental evidence of broad permissiveness of this insertion site.

In addition, the HA₀ influenza A epitope can be combined with M2e in a similar tandem fashion. Other influenza virus epitopes, such as virus neutralizing epitopes from HA protein, or CTL epitopes can be inserted alone or in various combinations at this location (or by analogy at some other locations in NS1 or in other viral proteins), including together with M2e.

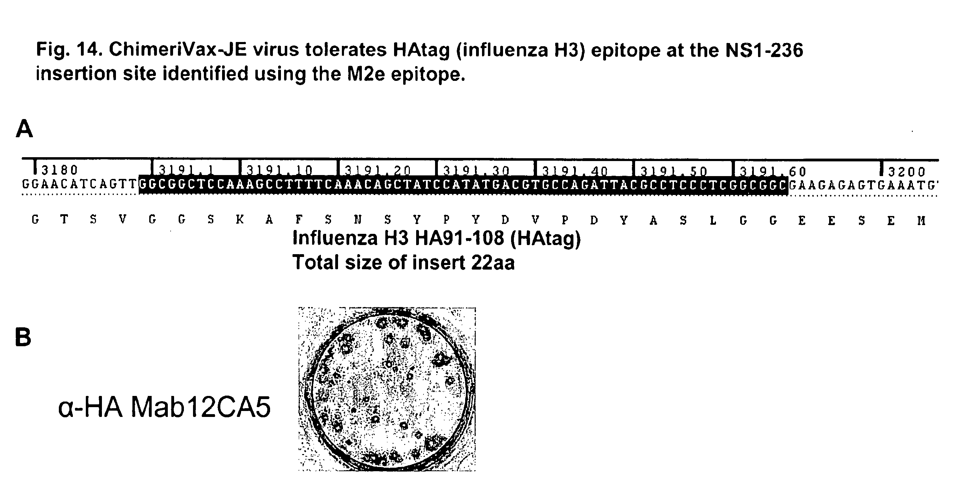

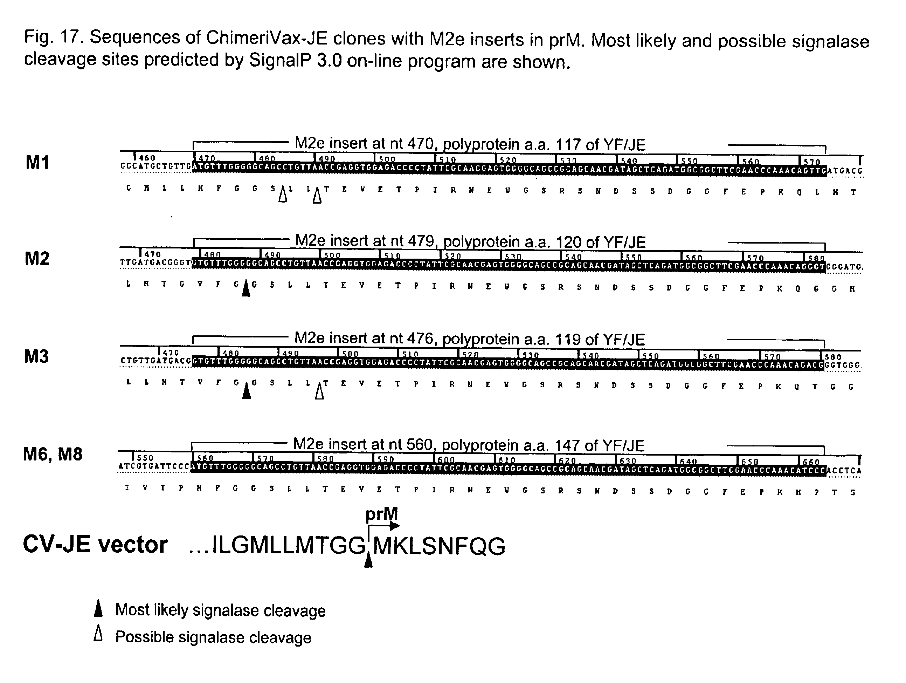

To further demonstrate broad permissiveness of the NS1-236 insertion site, the SKAFSNCYPYDVPDYASL linear protective epitope of influenza H3 virus (also referred to as HAtag epitope), which can provide protection against various H3 influenza strains (Bui et al., Proc. Natl. Acad. Sci. U.S.A. 104:246-251, 2007), was engineered after the NS1-236 residue, and recombinant virus was generated using the standard two-plasmid method. The epitope was flanked by two Gly residues at both sides for flexibility, and its Cys residue was changed to Ser. The insert sequence of the recovered viable virus is shown in Fig. 17A. Plaques of the virus (Vero cells) were stained with anti-HAtag MAb 12CA5 (Fig. 14B). Thus, the NS1-136 insertion site found by random insertion of M2e epitope is permissive for epitopes (e.g., HAtag) having totally different sequence. Similar to M2e insertion, this example also demonstrates insertion of not only of a B-cell epitope, but also a T-cell epitope, since HAtag represents both a B-cell as well as a T-cell influenza virus epitope (Bui et al., Proc. Natl. Acad. Sci. U.S.A. 104:246-251, 2007).

### Genetic stability, and growth kinetics in cell culture of ChimeriVax™-JE-NS1/M2e virus

The NS1 gene of Clone A25 virus (Fig. 6, panel A), which had the highest titer of 7 log₁₀ pfu/mL at passage 2 (P2; the research viral stock produced following 3 cycles of plaque purification and two amplification passages), was used for further biological characterization. The efficient expression of M2e is additionally illustrated in Fig. 6D by immunofluorescence of A25 infected cells that were specifically stained with M2e MAb (as well as JE antibodies).

To determine whether the virus is genetically stable in vitro, it was passaged 10 times, to the P12 level, at an estimated MOI of 0.001 pfu/mL in Vero cells certified for vaccine production. When P12 virus was stained in an immunofocus assay with M2e MAb or JE HIAF, all plaques stained with both antibodies and yielded the same titer of 8 log₁₀ pfu/mL (Fig. 6B). This demonstrated that the virus at passage 12 stably maintained its insert.

Some Vero cell adaptation occurred during passages since virus became progressively more cytopathic, and plaques at P12 level were larger than plaques of the virus at P2. The average diameter of P12 virus plaques became comparable to that of ChimeriVax™-JE vector virus. When the full genome of the P12 virus was sequenced, eight nucleotide changes were detected (Table 4). Four changes resulted in amino acid substitutions: Val to Ala in the E protein at residue E-357, Met to Val at NS4B-95, and 2 substitutions immediately upstream from the M2e peptide (Ser to Leu at NS1-235, and Phe to Leu at residue 1ᵢₙₛ). Some of the latter adaptations must have been responsible for increased plaque size and better virus replication (see below). None of these changes are reversions of attenuation markers in the ChimeriVax™-JE vaccine. (Mutations in three other clones, A11, A79, and A88, which were also passaged to P12 and sequenced, and were found to stably maintain the M2e insert, are also shown in Table 4.)

Growth kinetics of the A25 clone at P2 and P12 levels were compared to ChimeriVax™-JE parental vector virus in Vero cells. The result of one representative experiment (MOI 0.001) is shown in Fig. 6C. P2 virus grew efficiently, but somewhat slower than ChimeriVax™-JE, peaking on day 6, one day later compared to the vector virus. In contrast, P12 virus peaked on day 5 at a titer higher than ChimeriVax™-JE, in the excess of 7 log₁₀ pfu/mL. The more efficient replication of P12 virus was more pronounced at MOI of 0.1. Thus, the A25 clone replicated more efficiently after 10 passages in Vero cells. Some of the sequence changes found at P12 may be beneficial for high yield manufacturing of recombinant vaccine virus.

### A pilot experiment using ChimeriVax™-JE-NS1/M2e A25 virus to establish a mouse model for analysis of immunogenicity and protective efficacy of ChimeriVax™-JE/flu recombinants

As with any viral vaccine vector, particularly one for which rodents are not natural hosts (e.g., natural hosts of YF, the wild type prototype of YF 17D, are monkeys and humans), the establishment of a relevant and useful small animal model is challenging. With such a model, it should be possible to compare the relative immunogenicity of multiple recombinant viral constructs expressing foreign antigens in various configurations. In order to determine an optimal route of immunization and to obtain preliminary evidence of immunogenicity for ChimeriVax™-JE-NS1/M2e, groups of 5-week-old Balb/c mice (N=10) were immunized subcutaneously (SC) or intraperitoneally (IP) with 5 log₁₀ pfu/dose of the A25 clone (groups 1 and 2, respectively; Table 5). A positive control group 3 received SC dose of 10 µg of hepatitis B core particles containing M2e peptide on the surface produced in E. coli (HBc-M2e; Fiers et al., Virus Res. 103:173-176, 2004) with alum adjuvant; this group was similarly boosted on day 20. Negative control groups 4 and 5 were immunized SC with ChimeriVax™-JE vector (5 log₁₀ pfu), or mock-immunized (diluent).

Viremia in individual animals in groups inoculated with viruses was determined in sera collected on days 1, 3, 7, 9, and 11. The A25 virus caused no detectable viremia by either route. Two out of 10 animals inoculated with ChimeriVax™-JE virus had low-level viremia (50 and 275 pfu/mL) on day 1 only, which most likely represented the inoculated virus. Thus, the A25 virus failed to cause pronounced systemic infection by both routes.

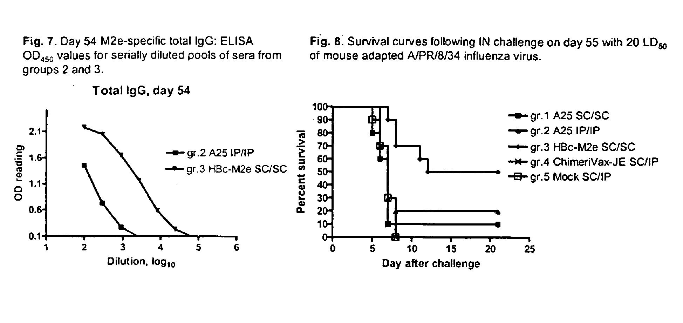

On day 38, all animals were bled and anti-M2e antibody responses were determined by ELISA in pools of sera for each group. In virus-immunized groups, low-level responses were only detected in group 2 (A25 IP), which had total IgG and IgG2a titers of 100 (and no detectable IgG1), while titers in group 3 (HBc-M2e SC/SC) were high, as expected: 218,700, 218,700, and 24,300 for total IgG, IgG1, and IgG2a, respectively. For this reason, groups 1, 2, and 4 were boosted on day 40 with 5 log₁₀ pfu of respective viruses: group 1 was boosted SC, while the other groups, IP. (Group 5 also received an IP dose of diluent.) Two weeks later (day 54), animals were bled again and M2e antibody responses were measured in pools of sera (Table 5). The A25 virus boost resulted in a dramatic increase in antibody titers in group 2 (A25 IP/IP). Total IgG titer in this group increased approximately 30-fold to 2,700. In group 3 (HBc-M2e), total IgG titer was 72,900. The 450 nm OD readings for total IgG are illustrated for groups 2 and 3 in Fig. 7. Importantly, while HBc-M2e immunization resulted in predominantly IgG1 response, nearly all antibodies induced by A25 virus were of the IgG2a subclass (Table 5). IgG2a antibodies are the main mediators of ADCC, which is considered to be the principal mechanism of M2e-induced protection from influenza infection. Thus, an efficient mouse model for measuring immunogenicity of ChimeriVax™-JE/flu recombinants has been established relying on IP immunization followed by IP boost.

On day 55, animals were challenged intranasally (IN) with a high dose of 20 LD₅₀ of mouse-adapted A/PR/8/34 influenza virus. This dose is 5 times higher compared to the standard challenge dose of 4 LD₅₀ used in HBc-M2e studies. In this pilot experiment, we deliberately chose the high dose to answer the question of whether more efficient protection is possible, as compared to HBc-M2e immunization, when M2e is delivered by ChimeriVax™-JE viral vector, even if post-immunization M2e antibody titers are lower. Theoretically, this could be due to non-specific viral stimulation of antigen presenting cells, CTL response (M2e peptide contains a CTL epitope), induction of robust T cell help, as well as some mechanisms of innate immunity. Postchallenge survival curves are shown in Fig. 8. As expected given the challenge dose, survival in HBc-M2e immunized animals was incomplete (50%). Two animals survived in group 2 immunized IP/IP with A25 virus, which had the highest M2e antibody titers among the two A25-immunized groups (20% survival). One animal survived in group 1 (A25 SC/SC). All animals in the negative control groups 4 and 5 died. From these data, there appears to be a clear correlation between the level of protection and M2e antibody titer, irrespective of whether animals are immunized with a recombinant virus or a subunit vaccine. However, it should be noted that some of the above mechanisms may have played a role in A25 immunization, as the actual µg amount of M2e delivered to mice by the virus is unknown and may have been very low due to limited replication of the virus in this model. This aspect can be addressed in the hamster model in which more efficient peripheral virus replication is expected. In primates/humans, ChimeriVax™-JE (as well as other ChimeriVax™ and YF 17D vaccines) causes a relatively efficient systemic infection with peak viremia titers of ∼ 2 log₁₀ pfu/M1. Thus, a robust M2e response and protection from influenza after a single inoculation of virus at a relatively low dose is expected.

### Mouse experiment 2 using A25 virus

An additional mouse experiment was done with the A25 virus using younger, 4-week-old mice (from two vendors), and a higher IP dose of A25 virus (7 log₁₀ pfu/ml). The experiment design is shown in Table 6. In most groups the A25 P2 virus stock was used (which was also used in the previous experiment); this experiment also included one group (#5) inoculated with the Vero cell-adapted A25 P12 virus described above. Negative controls were ChimeriVax-JE and diluent (groups 2, 4, and 7). Positive control Taconic mice were inoculated SC with HBc-M2e particles (referred to as Acam-Flu-A) mixed with alum adjuvant. Among Jackson mouse groups, there were two groups created to test for synergistic effect between A25 virus and Acam-Flu-A: group 8 received only Acam-Flu-A without adjuvant via the IP route, and group 9 received Acam-Flu-A mixed with A29, also IP. All mice were boosted at 1 month after initial inoculation, and M2e-specific antibody titers (total IgG, and IgG1, IgG2a, IgG2b, and IgG3 types) were determined on day 59 by ELISA in individual sera (for total IgG) or in pools of sera for each group (for IgG isotypes); M2e-specific total IgG titers were also determined on day 30 (before boost). ELISA titers are shown in Table 7; GMT values are given for total IgG determined in individual sera. The data were in agreement with the previous mouse experiment, except that A25 immunized animals had significantly higher M2e peptide-specific antibody titers. Most A25 and Acam-Flu-A inoculated animals seroconverted after the first dose, on day 30. On day 59 (∼ 1 month after boost) all animals in A25 and Acam-Flu-A groups were seropositive and total IgG titers increased dramatically compared to day 30. As expected, Acam-Flu-A/alum adjuvant immunization (group 3) resulted in predominantly Th2 type response, with IgG1 titers being the highest compared to the other IgG isotypes. Immunization with A25 (groups 1, 5, and 6) resulted in predominantly Th1 type response associated with higher IgG2a titers, which is the desired type for M2e-mediated protection via the ADCC mechanism; and IgG2b and IgG3 antibodies that have been also implicated in ADCC (Jegerlehner et al., J. Immunol. 172:5598-5605, 2004) were detected. This again demonstrated high immunogenicity of the M2e epitope inserted at the NS 1-236 site of ChimeriVax-JE.

An important observation in this experiment was that co-inoculation of Acam-Flu-A with A25 virus significantly increased the anti-M2e antibody response as compared to inoculation of Acam-Flu-A or A25 virus alone (compare groups 9 with groups 8 and 6 in Table 7). On day 59, total IgG GMTs were 95,940 and 35,050 for groups 9 and 8, respectively (the proportional difference was even more pronounced on day 30). Thus, a strong synergistic effect of co-inoculation was observed. Moreover, while Acam-Flu-A alone induced mostly Th1 type response (titers of IgG1, IgG2a, IgG2b, and IgG3 of 72,900, 8,100, 300, and 900, respectively), co-inoculation of Acam-Flu-A with A25 virus led to a clear Th2 shift as evidenced by a lower proportion of IgG1 and a significantly higher proportion of the other antibody isotypes (titers of 72,900, 72,900, 8,100, and 8,100 for IgG1, IgG2a, IgG2b, and IgG3, respectively). The synergistic effect cannot be attributed solely to the increase of antigen (M2e) mass by A25 virus in co-inoculated animals, since A25 inoculation alone resulted in a modest immune response (in Jackson balb/c mice, see group 6 in Table 7). These effects could be also due to an adjuvant effect of replication of the virus, e.g., in dendritic cells in the inoculation site. Such adjuvant effects have been reported for alphavirus replicons (Thompson et al., Proc. Natl. Acad. Sci. U.S.A. 103:3722-3727,2006; Hidmark et al., J. Virol. 80:7100-7110,2006).

### Expression of M2e randomly inserted in the E protein of ChimeriVax-JE

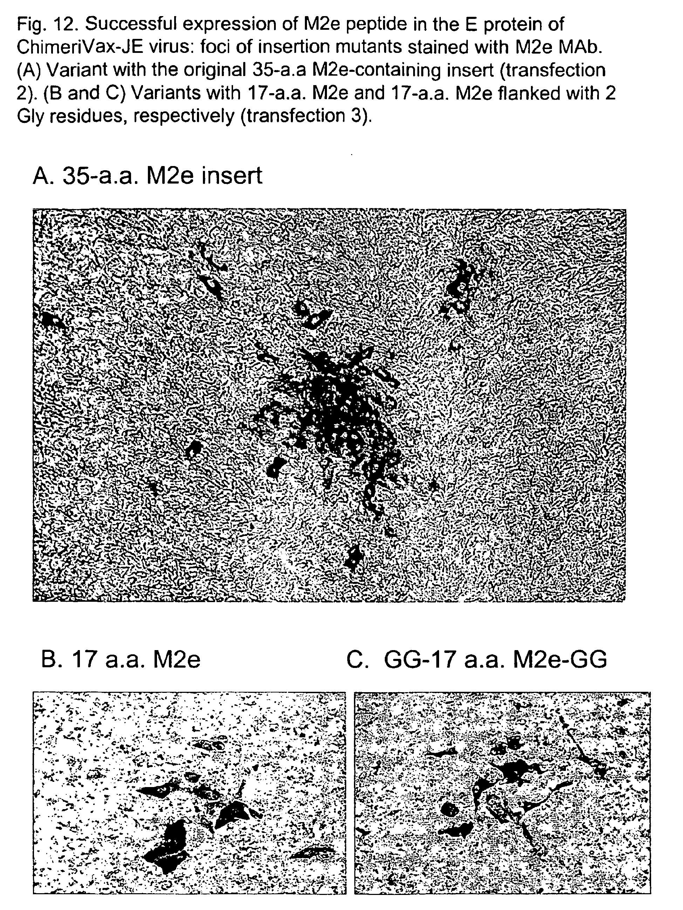

Three experiments were done to determine whether M2e can be randomly inserted and expressed in the E protein of ChimeriVax-JE vector, on the surface of viral particles. In the first experiment, RNA was synthesized with SP6 RNA polymerase on the pBSA-AR2-rM2e plasmid library (Step 16 in Fig. 2). Vero cells were transfected with the RNA using lipofectamine. Only non-mutant virus plaques were observed in harvested cell supernatants, which were not stained with M2e MAb. Presumably, as in the case with random insertion in NS1, virus not bearing M2e insert quickly appeared due to insertions at unstable locations in E, and became dominant. In the second experiment, the E-M2e gene library was extracted from pUCAR02-rM2e (Step 13 in Fig. 2) with NsiI and KasI, and in vitro ligated into the pBSA-AR2stop vector (from Step 15, Fig. 2). The ligation product was linearized with XhoI and transcribed in vitro. Vero cells were electroporated with the synthesized RNA, the transfected cell suspension was then serially diluted (to reduce interference between nonmutant and M2e-positive viruses), and the cell dilutions were plated in Petri dishes. Untransfected Vero cells were added to dishes seeded with higher dilutions of transfected cells on order to ensure that cell monolayers were confluent. After attachment, cell monolayers were overlaid with agar. When monolayers were stained 6 days later with M2e Mab (after removal of agarose overlay), several positive foci were observed at higher transfected cell dilutions (1:4 and 1:8). An example of one of the foci is shown in Fig 12A. The number of foci and their sizes were smaller compared to some of those observed with NS1-M2e library transfections, indicating that it may be more difficult to insert the 35-amino acid long insert (used in pUC-AR02-rM2e; the same as in Fig. 5) into the E protein compared to NS1. In the third experiment, a shorter M2e insert (SLLTEVETPIRNEWGSR) was produced by annealing two complementary phosphorylated primers. The nucleotide sequence of the insert is as follows:

5'-P-AGC CTT CTA ACC GAG GTC GAA ACG CCT ATC AGA AAC GAA
TGG GGG AGC AGA-3'

The same insert but containing two extra Gly linker residues on both sides, for flexibility (total length 21 amino acids), was similarly produced. The nucleotide sequence of the second insert as follows:

5'-P-GGA GGA AGC CTT CTA ACC GAG GTC GAA ACG CCT ATC AGA
AAC GAA TGG GGG AGC AGA GGC GGC-3'

The two inserts were ligated into the blunt PmeI site of pUC-AR02-rTn7enr library (Step 8, Fig. 2) in place of the transprimer. The vector plasmid DNA was dephosphorylated before ligation. Two new plasmid libraries were produced, pUC-AR2-17M2e and pUC-AR2-17gM2e, respectively. The NsiI-KasI inserts of the two libraries were transferred to the pBSA-AR2stop vector, resulting in pBSA-AR2-17M2e and pBSA-AR2-17gM2e full-length libraries, which were then used for in vitro transcription. The two latter libraries were first digested with PmeI to eliminate any full-length template DNA molecules not containing the inserts (while in insert-containing molecules, the Pmel cloning sites on both sides of the insert are ablated). Then they were linearized with XhoI and transcribed with SP6 RNA polymerase. Vero cells were electroporated with the transcripts and seeded, undiluted, into Petri dishes and overlaid with agarose after cells attached. To avoid interference with insert-less virus, the monolayers were stained with M2e Mab early, on day 4 post-transfection. Up to ∼ 100 small foci were observed in the two transfections. Examples of such foci are shown in Fig. 12A and B, for ChimeriVax-JE viruses containing the 17-amino acid M2e insert in the E protein, and the GG-17 amino acid -GG insert, respectively. Thus, it appears that shortening the insert from 35 amino acids to 17 or 21 amino acids significantly increased recovery of recombinant viruses. It is possible that some of the observed M2e-positive variants, once isolated, will replicate reasonably well. If necessary, more efficiently replicating variants can be isolated from additional transfections. In addition, slowly replicating variants can be serially passaged in, e.g., Vero cells, with the expectation that some second site mutation(s) will occur improving growth. This example clearly demonstrates the possibility of randomly inserting foreign immunologic epitopes into the E protein.

### Random insertion of M2e epitope in the prM protein of ChimeriVax-JE vector virus

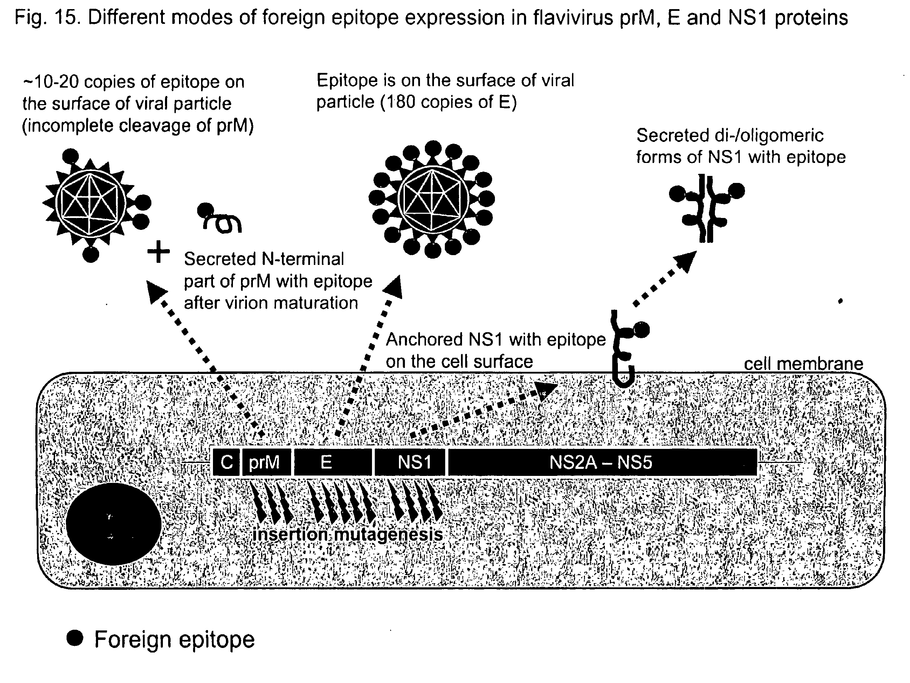

The different modes of expression in viral glycoproteins (prM, E, or NS1) are illustrated in Fig. 15. Epitopes inserted into the E protein will be presented on the surface of viral particles (180 copies) and therefore can be expected to be the most immunogenic. Expression in the NS1 protein delivers the inserted epitope to the surface of infected cells, as well as extracellularly in the secreted NS1 oligomers. Although high immunogenicity of the later mode was demonstrated in experimental examples above, it may be lower in this case compared to expression in E (still sufficiently high for some epitopes, e.g., virus-neutralizing antibody epitopes providing much stronger protection compared to non-neutralizing epitopes, such as M2e of influenza). Expression in prM will result in partial presentation on the surface of viral particles due to the known phenomenon of incomplete cleavage of prM by furin in the process of flavivirus particle maturation, and possibly in additional extracellular presentation within the secreted N-terminal part of prM generated by furin cleavage. This mode of expression is also expected to be highly immunogenic, more immunogenic than expression in NS 1. If epitopes can be inserted in the mature M protein (C-terminal portion of prM), all epitope molecules may be also presented on the surface of viral particle (180 copies), similar to expression in E.

To insert the M2e epitope (35 amino acids total length of insert) into prM of ChimeriVax-JE, between SphI and NsiI sites (SphI is located upstream from the start of prM gene), pBSA-AR1-rM2e plasmid library was constructed (Fig. 2). The representativeness of this library was ∼ 10⁵ colonies. It was used as template for in vitro transcription, and the resulting RNA transcripts were used to transfect Vero cell monolayers with lipofectamine. Transfected cells were overlaid with agarose and cell monolayers were stained with M2e MAb on day 5-6. M2e-positive plaques were observed. M2e-positive viral clones corresponding to positive plaques were harvested from the agarose overlay and further purified in additional rounds of plaque purification, followed by 2 amplification passages to prepare 5 pure viral stocks designated M1, M2, M3, M6, and M8.

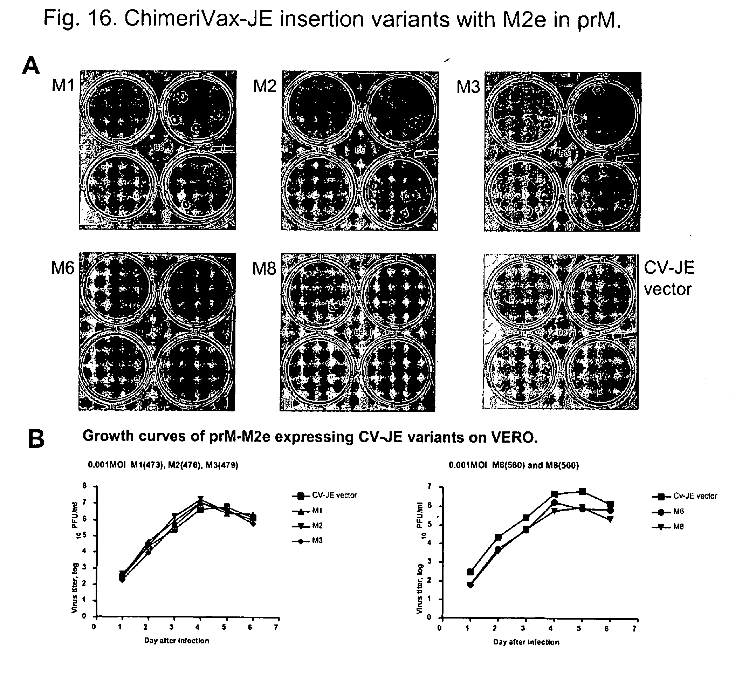

All new recombinant clones were efficiently stained with M2e and JE antibodies, while ChimeriVax-JE vector virus plaques were stained with JE antibodies only. Examples of plaques stained on day 5 in standard plaque assay (methyl cellulose overlay) are shown in Fig. 16A. Plaques of M1, M2, and M3 insertion mutants were larger compared to ChimeriVax-JE, while plaques of M6 and M8 clones were smaller. (This difference in plaque sizes was more pronounced under agarose overlay.) Thus, it appears that the M1-3 clones were able to replicate better in vitro compared to the vector virus. This was confirmed in growth curve experiment (Fig. 16B). M1-3 clones grew faster and produced higher peak titers than ChimeriVax-JE, while titers of M6 and M7 were slightly lower.

Insertion locations were determined in the clones by sequencing. The results are shown in Fig. 17. Interestingly, the M2e insert was added to the very N-terminus of the JE-specific prM of ChimeriVax-JE virus in clones M1, M2, and M3, although at different amino acids. The location in clones M6 and M8 was the same (after Pro residue 147 in the viral ORF; or prM-26). In ChimeriVax-JE virus the N-terminus of prM (MKLS...) is formed by host cell signalase cleavage (Fig. 17). In clones M1, M2, and M3, the insert was incorporated 4, 1, and 2 amino acid residues upstream from the beginning of JE prM, respectively. Thus in these viruses the N-termini of mutant prM contain the M2e peptide sequences followed by 4, 1, or 2 viral residues preceding native prM sequence, followed by the prM sequence. New signalase cleavage sites in the mutants were predicted with the common SignalP 3.0 on-line program using two different algorithms (shown in Fig. 17). In M1 clone, the two possible cleavages may remove one or three N-terminal amino acids of M2e. In M2, the strongly predicted, single cleavage will result in N-terminal Gly followed by complete M2e sequence. In M3, the N-terminus will either as in M2 or three of the M2e residues may be cleaved off by an alternative possible cleavage). The fact that plaques of the three clones were efficiently stained with M2e MAb suggests that cleavages in M1 and M3 occurred with minimum loss of M2e residues. Importantly, predicted probabilities of signalase cleavage for the M1-3 clones were higher compared to ChimeriVax-JE (e.g., 0.387 for M2 clone vs. 0.073 for ChimeriVax-JE). This may explain why the M1-3 viruses grow better than ChimeriVax-JE parent.

Thus, the prM protein is highly permissive for insertions at various locations, particularly its N-terminal residues. Based on the described results (larger plaques, more efficient replication in Vero cells, higher predicted signalase cleavage probability in M1-3 clones), we believe that the N-terminus of prM, which appears to be unimportant for flavivirus particle assembly, will be a broadly permissive insertion site and will tolerate various other inserts, including long inserts (e.g., 50, 100, 200, 400 amino acids, etc.). We thus are inserting at this location HIV gag, peptides comprising up to 200 first residues of HPV 16 L2 protein, influenza HA₁, and full-length HA (∼550 a.a. in length). These are designed to contain heterologous sequences fused with the N-terminus or prM (as is the case with M2e in M1-3 clones), or to be cleaved off from prM by incorporation of additional signal, or an appropriate protease cleavage site, or autoprotease, in front of vector virus prM sequence.

### Construction of a ChimeriVax-WN analog of the A25 virus (ChimeriVax-JE with M2e insertion at NS1-236)

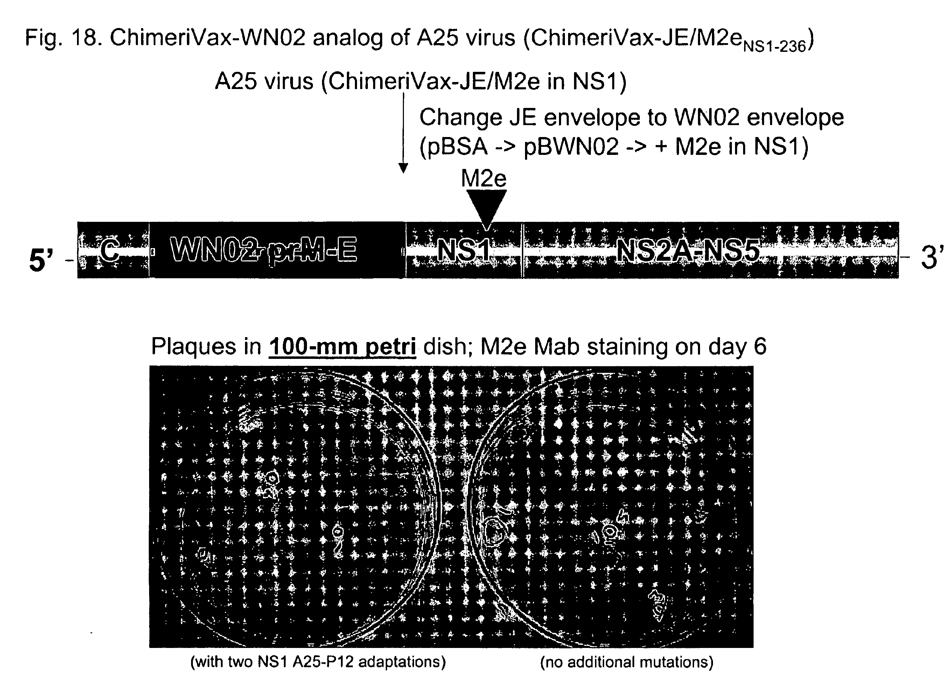

ChimeriVax-JE virus, as well as the A25 virus described above, do not replicate efficiently in mice (e.g., there is no detectable postinoculation viremia). Nevertheless, ChimeriVax-JE replicates better in humans (∼ 2 log₁₀ pfu/ml viremia) (Monath et al., J. Infect. Dis. 188:1213-1230, 2003), and thus A25 virus could induce a high M2e antibody response in humans and protect them from influenza infection. We recently demonstrated that ChimeriVax-WN virus (the WN02 human vaccine version; WO 2004/045529) replicates very well in hamsters (∼ 3 log₁₀ pfu/ml viremia) (WO 2006/116182 A1), as well as in humans (∼ 2 log₁₀ pfu/ml viremia) (Monath et al., Proc. Natl. Acad. Sci. U.S.A. 103:6694-6699, 2006). In order to obtain additional evidence of protection by the M2e epitope expressed at the NS1-236 site of ChimeriVax viruses using a more robust model (ChimeriVax-WN02 in hamsters vs. ChimeriVax-JE in mice), a ChimeriVax-WN02/M2e_{NS1-236} analog of the A25 virus was constructed. The JE-specific prM-E genes in the pBSA plasmid containing full-length ChimeriVax-JE cDNA were replaced with prM-E genes of ChimeriVax-WN02 virus, using standard cloning techniques. This resulted in pBWN02 plasmid (Fig. 18). The NS1 gene with M2e insert from A25 virus (with or without two Vero cell adaptations right upstream from the M2e sequence; Table 4) was cloned into pBWN02. The resulting two plasmids were transcribed in vitro, and Vero cells were transfected with the RNA transcripts and overlaid with agar. Very large plaques were observed on day 6, which were stained with M2e MAb (Fig. 18, bottom panel).

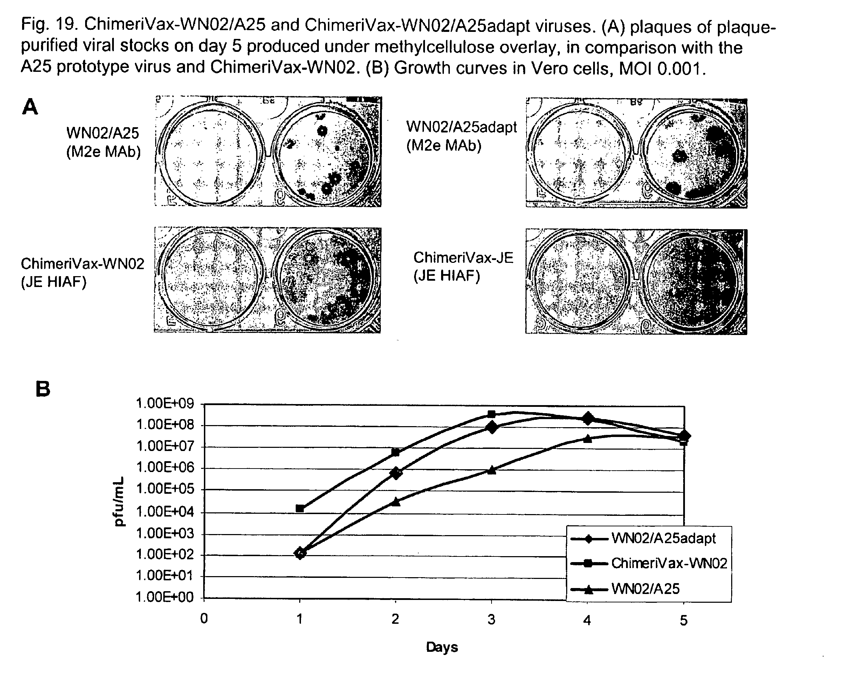

The two versions of ChimeriVax-WN02/M2e_{NS1-236}, WN02/A25 and WN02/A25adapt, were plaque-purified once and stocks of cloned viruses were prepared by additional amplification in Vero cells. Examples of plaques in comparison with ChimeriVax-WN02 and ChimeriVax-JE are shown in Fig. 19A. Growth curves of the new viruses in Vero cells are shown in Fig. 19B. The WN02/A25 virus grew somewhat less well than ChimeriVax-WN02 (peak titer ∼ 7.5 log₁₀ pfu/ml vs. ∼8.7 log₁₀ pfu/ml, respectively). Similar to the adapted A25 virus (see in Fig. 6C), the WN02/A25adapt version (with two amino acid changes upstream from M2e sequence) grew better, almost as well as ChimeriVax-WN02. Thus, an insertion originally introduced into NS1 protein of ChimeriVax-JE was successfully transferred to ChimeriVax-WN02 vaccine virus. The two cell culture adaptations originally observed in A25 virus enhanced growth of WN02/A25 virus.

### Conclusion

In conclusion, we successfully performed transposon-mediated mutagenesis of the prM/M, E, and NS.1 genes of ChimeriVax™-JE vaccine virus to randomly insert the consensus M2e protective epitope of influenza A virus with the purpose of generating a highly effective universal vaccine against influenza A. Feasibility of the method was demonstrated by quickly producing a number of virus mutants containing the insert, recognizable by anti-M2e antibody, in the prM and NS1 proteins, and inserting M2e peptide into the E protein. We also showed that the A25 clone of ChimeriVax™-JE-NS1/M2e virus and several clones of ChimeriVax™-JE-prM/M2e virus replicated efficiently in Vero cells, and the M2e insertion sites in these viruses were identified. Also, we showed that the A25 virus is genetically stable, as it has maintained the M2e insert for 10 low-MOI passages in vitro. Some insertion sites identified by the direct random mutagenesis approach of the invention can be broadly permissive, both in terms of insert size and sequence, as was exemplified using the NS1-236 location. Permissive insertion sites found in one flavivirus can be used in other flavivirues, as exemplified in our experiments by transferring NS1 gene with M2e insertion from ChimeriVax-JE to ChimeriVax-WN. Further, an efficient IP prime/IP boost model for analysis of immunogenicity in mice was successfully established, and high immunogenicity of one insertion variant was demonstrated. Despite undetectable peripheral replication in mice, including after IP inoculation, the virus was highly immunogenic and induced predominantly IgG2a M2e antibodies, which is highly desirable in terms of ADCC-mediated protection by M2e immunization. Another novel finding in our experiments was a strong synergistic effect of co-inoculation of a viral recombinant expressing M2e peptide with a subunit M2e-based vaccine candidate.

As discussed above, the method described herein is applicable to all other ChimeriVax™ target proteins, as well as other live vaccine viruses as vectors, including YF17D or non-flavivirus live vaccines, or non-viral vector organisms. This approach can be used to construct recombinant vaccines against a wide range of pathogens of human public health and veterinary importance.

Note that the outlined sequence of construction steps (Fig. 2) can vary and still be within the scope of this invention. Also, transposons other than Tn7 may be used for random insertion of a random restriction site or a foreign epitope directly. The latter, as well as using restriction sites other than PmeI for random insertion, or different selective markers at any of the construction steps, or using any different methods to isolate viable mutant viruses (e.g., ELISA using supernatants from virus infected cells, or cell sorting to isolate positive cells, etc.) or to characterize viruses in vitro and in vivo, etc., do not change the meaning of this invention.

**Table 1 - List of examples of pathogens from which epitopes/antigens/peptides can be derived**

| VIRUSES: |
|---|
| Flaviviridae |
| Yellow Fever virus |
| Japanese Encephalitis virus |
| Dengue virus, types 1, 2, 3 & 4 |
| West Nile Virus |
| Tick Borne Encephalitis virus |
| Hepatitis C virus (e.g., genotypes 1a, 1b, 2a, 2b, 2c, 3a, 4a, 4b, 4c, and 4d) |
| Papoviridae: |
| Papillomavirus |
| Retroviridae |
| Human Immunodeficiency virus, type I |
| Human Immunodeficiency virus, type II |
| Simian Immunodeficiency virus |
| Human T lymphotropic virus, types I & II |
| Hepnaviridae |
| Hepatitis B virus |
| Picornaviridae |
| Hepatitis A virus |
| Rhinovirus |
| Poliovirus |
| Herpesviridae: |
| Herpes simplex virus, type I |
| Herpes simplex virus, type II |
| Cytomegalovirus |
| Epstein Barr virus |
| Varicella-Zoster virus |
| Togaviridae |
| Alphavirus |
| Rubella virus |
| Paramyxoviridae: |
| Respiratory syncytial virus |
| Parainfluenza virus |
| Measles virus |
| Mumps virus |
| Orthomyxoviridae |
| Influenza virus |
| Filoviridae |
| Marburg virus |
| Ebola virus |
| Rotoviridae: |
| Rotavirus |
| Coronaviridae |
| Coronavirus |
| Adenoviridae |
| Adenovirus |
| Rhabdoviridae |
| Rabiesvirus |
| |

| BACTERIA: |
|---|
| |
| Enterotoxigenic *E*. *coli* |
| Enteropathogenic *E. coli* |
| *Campylobacter jejuni* |
| |
| *Helicobacter pylori* |
| |
| *Salmonella typhi* |
| |
| *Vibrio cholerae* |
| |
| *Clostridium difficile* |
| *Clostridium tetani* |
| *Streptococccus pyogenes* |
| *Bordetella pertussis* |
| *Neisseria meningitides* |
| *Neisseria gonorrhoea* |
| *Legionella neumophilus* |
| |
| Clamydial spp. |
| Haemophilus spp. |
| Shigella spp. |
| |

| PARASITES: |
|---|
| |
| Plasmodium spp. |
| Schistosoma spp. |
| Trypanosoma spp. |
| Toxoplasma spp. |
| Cryptosporidia spp. |
| Pneumocystis spp. |
| Leishmania spp. |

**Table 2 - Examples of select antigens from listed viruses**

| VIRUS | ANTIGEN |
|---|---|
| Flaviviridae | |
| Yellow Fever virus | Nucleocapsid, M & E glycoproteins |
| Japanese Encephalitis virus | " |
| Dengue virus, types 1, 2, 3 & 4 | " |
| West Nile Virus | " |
| Tick Borne Encephalitis virus | " |
| | |
| Hepatitis C virus | Nucleocapsid, E1 & E2 glycoproteins |
| | |
| Papoviridae: | |
| Papillomavirus | L1 & L2 capsid protein, E6 & E7 transforming protein (oncopgenes) |
| | |
| Retroviridae | |
| Human Immunodeficiency virus, type I | gag, pol, vif, tat, vpu, env, nef |
| Human Immunodeficiency virus, type II | " |
| Simian Immunodeficiency virus | " |
| Human T lymphotropic virus, types I & II | gag, pol, env |

**Table 3 - Examples of B and T cell epitopes from listed viruses/antigens**

| VIRUS | ANTIGEN | EPITOPE | LOCATION | SEQUENCE (5'-3') |
|---|---|---|---|---|
| Flaviviridae | | | | |
| Hepatitis C | Nucleocapsid | CTL | 2-9 | STNPKPQR |
| | | | 35-44 | YLLPRRGPRL |
| | | | 41-49 | GPRLGVRAT |
| | | | 81-100 | YPWPLYGNEGCGWAGWLLSP |
| | | | 129-144 | GFADLMGYIPLVGAPL |
| | | | 132-140 | DLMGYIPLV |
| | | | 178-187 | LLALLSCLTV |
| | | | | |
| | E1 glycoprotein | CTL | 231-250 | REGNASRCWVAVTPTVATRD |
| | | | | |
| | E2 glycoprotein | CTL | 686-694 | STGLIHLHQ |
| | | | 725-734 | LLADARVCSC |
| | | | 489-496 | CWHYPPRPCGI |
| | | | 569-578 | CVIGGVGNNT |
| | | | 460-469 | RRLTDFAQGW |
| | | | 621-628 | TINYTIFK |
| | | | | |
| | | B cell | 384-410 | ETHVTGGNAGRTTAGLVGLL TPGAKQN |
| | | | 411-437 | IQLINTNGSWHINSTALNCNESLNTG W |
| | | | 441-460 | LFYQHKFNSSGCPERLASCR |
| | | | 511-546 | PSPVVVGTTDRSGAPTYSWGANDTDV FVLNNTRPPL |
| | | T helper | 411-416 | IQLINT |
| | | | | |

| Papoviridae | | | | |
|---|---|---|---|---|
| HPV 16 | E7 | T helper | 48-54 | DRAHYNI |
| | | CTL | 49-57 | RAHYNIVTF |
| | | B cell | 10-14 | EYMLD |
| | | | 38-41 | IDGP |
| | | | 44-48 | QAEPD |
| | | | | |
| HPV 18 | E7 | T helper | 44-55 | VNHQHLPARRA |
| | | | 81-90 | DDLRAFQQLF |

**Table 4. Genetic stability of Clone A25 of ChimeriVax-JE-NS1/M2e virus, as well as clones A11, A79, and A88. Full genomes of viruses were sequenced at P12 genetic stability passage. Nucleotide changes/heterogeneities and a.a. changes are shown.**

| **Gene** | **Nt (a.a.) position** | **A25** | **A11** | **A79** | **A88** |
|---|---|---|---|---|---|
| **C** | 401 | | | | |
| **M** | 931 | T-C | | | |
| | 935 (60) | | C(R)-T(C) | | |
| | 956 (67) | | | | C/G (L/V) |
| **E** | 1223 (81) | | | C/T (H/Y) | |
| | 1963 | C-A | | | |
| | 2052 (357) | T(V)-A(A) | | | |
| | 2165 (395) | | C(H)-T(Y) | | C/T (H/Y) |
| | 2453 (491) | | | C(L)-T(F) | |
| **NS1** | 3012(177) | | | | T/C (I/T) |
| | 3186 (235) | C(S)-T(L) | | | C/T (S/L) |

| **M2e Insert¹** | **Present? lᵢₙₛ (lᵢₙₛ)** | **yes T(F)-C(L)** | **yes** | **yes** | **Yes** |
|---|---|---|---|---|---|
| | 3375 (298) | | C(T)-T(I) | C(T)-T(I) | |
| **NS2a** | 3910 | G-A | | | |
| | 4099 | | C-T | | |
| | 4141 | | | T-C | |
| **NS3** | 5683 | G-A | | | |
| | 5938 | | | | A/G |
| | 6031 | | | C-T | |
| | 6043 | | T-C | | |
| **2K** | 6893 (16) | | | | A/G (T/A) |
| | 6906 (20) | | | | C/T (A/V) |
| **NS4b** | 7199 (95) | A(M)-G(V) | | | |
| **NS5** | 7963 | | | G/T | |
| | 8008 (114) | | | | G(M)-A(I) |
| | 8059 | | | | T/C |
| **3'UTR** | 10689 | | | | G-T |

| | | | | | |
|---|---|---|---|---|---|
| ¹The location of the insert in NS1 and nt/a.a. numbering shown in Fig. 5. | | | | | |

**Table 5. M2e antibody responses in Balb/c mice on day 54 (2 weeks after boost of groups 1, 2, 4, and 5)¹.**

| Group | Immunized | | Boost² | Day 54 M2e antibody titers | | |
|---|---|---|---|---|---|---|
| | With | Route | | total IgG | IgG1 | IgG2a |
| 1 | A25 | SC | SC | 100 | <100 | <100 |
| 2 | A25 | IP | IP | **2,700** | **300** | **2,700** |
| 3 | HBc-M2e | SC | SC | **72,900** | **72,900** | **24,300** |
| 4 | ChimeriVax-JE | SC | IP | <100 | <100 | <100 |
| 5 | Mock (diluent) | SC | IP | <100 | <100 | <100 |

| | | | | | | |
|---|---|---|---|---|---|---|
| ¹ For viruses, immunizing and boost doses were 5 log₁₀ pfu; for HBc-M2e, the doses were 10 µg of particles + alum. ² Group 3 was boosted on day 20, while groups 1, 2, 4 and 5 were boosted on day 40. | | | | | | |

**Table 6. Design of mouse experiment #2 using A25 virus (4 week-old female balb/c mice from two vendors). ELISA antibody titers were determined on day 59 (∼ one month after boost).**

| Group | Vendor | No. of animals | Inoculate | Dose | Prime Route | Boost Route (1 mo.) |
|---|---|---|---|---|---|---|
| 1 | Taconic | 8 | A25 P2 | 7 log | IP | IP |
| 2 | | 8 | CM-JE | 7 log | IP | IP |
| 3 | | 8 | Acam-Flu-A + Alum | 10 µg + alum | SC | SC |
| 4 | | 8 | Diluent | - | IP | IP |
| 5 | | 8 | A25 P12 | 7 log | IP | IP |
| 6 | Jackson | 8 | A25P2 | 7 log | IP | IP |
| 7 | | 8 | CV-JE | 7 log | IP | IP |
| 8 | | 3 | Acam-Flu-A | 10 µg | IP | |
| 9 | | 4 | A25P2 + Acam-Flu-A | 7 log/ 10µg | IP | IP |

**Table 7. M2e-specific antibody responses in mice in Experiment 2.**

| **Group** | **Inoculated with** | **Total IgG M2e ELISA on day 30 (pre-boost)** | | **M2e ELISA titers on day 59 (after boost)** | | | | |
|---|---|---|---|---|---|---|---|---|
| | | Serocon-verted | GMT³ | Total IgG GMT | Pooled IgG1 | Pooled IgG2a | Pooled IgG2b | Pooled IgG3 |
| 1 | A25P2 IP/IP | 8/8 | 1,004 | 10,090 | 8,100 | 24,300 | 900 | 2,700 |
| 2 | CV-JE IP/IP | 0/8 | <100 | < 100 | <100 | <100 | <100 | <100 |
| 3 | FluA/A1 SC/SC | 8/8 | 4,677 | >187,080 | 218,700 | 72,800 | 24,300 | 2,700 |
| 4 | Diluent IP/IP | N/D | N/D | N/D | N/D | N/D | N/D | N/D |
| 5 | A25P12 IP/IP | 6/8 | 155 | 3,695 | 300 | 8,100 | 100 | 300 |
| 6 | A25P2 IP/IP | 7/8 | 390 | 3,160 | 2,700 | 8,100 | 100 | 300 |
| 7 | CV-JE IP/IP | 0/8 | <100 | <100 | <100 | <100 | <100 | <100 |
| 8 | FluA IP/IP | 3/3 | 900 | 35,050 | 72,900 | 8,100 | 300 | 900 |
| 9 | A25P2/FluA IP/IP | 4/4 | 6,155 | 95,940 | 72,900 | 72,900) | 8,100 | 8,100 |

Use of singular forms herein, such as "a" and "the," does not exclude indication of the corresponding plural form, unless the context indicates to the contrary. Thus, for example, if a claim indicates the administration of "a" flavivirus, it can also be interpreted as covering administration of more than one flavivirus, unless otherwise indicated. Other embodiments are within the following claims.

## Claims

1. A method for generating a viral genome comprising a nucleic acid molecule encoding a heterologous immunogenic peptide, wherein the viral genome is the genome of a flavivirus, the method comprising the steps of:
(i) providing a target viral gene;
(ii) subjecting the target viral gene to mutagenesis to insert a restriction site;
(iii) ligating a nucleic acid molecule encoding a heterologous immunogenic peptide into the restriction site of the target viral gene to generate a mutant gene library;
(iv) transfecting cells with the viral genomes of step (iii) to initiate virus replication; and
(v) selecting viable virus recombinants with an antibody against the immunogenic peptide;
wherein the target viral gene is provided (a) in a shuttle vector and, after ligation of the nucleic acid molecule encoding the heterologous peptide into the site of mutagenesis of the target viral gene, the method further comprises the step of introducing the mutated target viral gene into a viral genome from which the target viral gene was derived, in place of the corresponding viral gene lacking the insertion, to generate viral genomes comprising insertions, or (b) in the context of an intact viral genome, and the method generates viral genomes comprising insertions.

2. The method of claim 1, wherein the shuttle vector comprises two or more target viral genes.

3. The method of claim 1 or 2, further comprising generating a viral vector from the viral genome comprising an insertion by introduction of the viral genome comprising an insertion into cells.

4. The method of claim 3, further comprising isolating the viral vector from the cells or the supernatant thereof.

5. The method of any of claims 1-4, wherein the mutagenesis step comprises introduction of one or more transprimers into the target viral gene by transposon mutagenesis.

6. The method of any of claims 1-5, further comprising the generation of a library of mutated viral genes.

7. The method of claim 1, wherein the viral genome is the genome of a chimeric flavivirus.

8. The method of any of claims 1-7, wherein the target viral gene is selected from the group consisting of genes encoding envelope, capsid, pre-membrane, NS1, NS2A, NSB, NS3, NS4A, NS4B, and NS5 proteins.

9. The method of any of claims 1-8, wherein the heterologous immunogenic peptide comprises a vaccine epitope

10. The method of claim 9, wherein the vaccine epitope is a B-cell epitope or a T-cell epitope.

11. A flavivirus comprising a genome in which at least one nucleic acid molecule encoding a heterologous immunogenic peptide is inserted within a sequence encoding a protein selected from the group consisting of pre-membrane protein and the nonstructural protein 1 (NS1), wherein the insertion in NS1 is between sequences encoding amino acids 236 and 237 thereof, and/or the insertion is at position -4, -2, or -1 preceding sequences encoding the capsid/pre-membrane cleavage site, or sequences encoding position 26 of the pre-membrane protein.

12. The flavivirus according to claim 11, further comprising at least one nucleic acid molecule encoding a heterologous immunogenic peptide inserted within a sequence encoding a protein selected from the group consisting of capsid, envelop, NS2A, NS2B, NS3, NS4A, NS4B and NS5.

13. The flavivirus of claim 11, wherein the flavivirus is a yellow fever virus, or a chimeric flavivirus.

14. The method of claim 7 or the flavivirus of claim 13, wherein the genome of the chimeric flavivirus comprises sequences encoding the capsid and non-structural proteins of a first flavivirus and the pre-membrane and envelope proteins of a second, different flavivirus.

15. The method or flavivirus of claim 14, wherein the first and second flaviviruses are independently selected from the group consisting of Japanese encephalitis, Dengue-1, Dengue-2, Dengue-3, Dengue-4, Yellow fever, Murray Valley encephalitis, St. Louis encephalitis, West Nile, Kunjin, Rocio encephalitis, Ilheus, Tick-borne encephalitis, Central European encephalitis, Siberian encephalitis, Russian Spring-Summer encephalitis, Kyasanur Forest Disease, Omsk Hemorrhagic fever, Louping ill, Powassan, Negishi, Absettarov, Hansalova, Apoi, and Hypr viruses.

16. The method of any of claims 1-9, 14 and 15 or the flavivirus of any of claims 11-15, wherein the heterologous peptide comprises an influenza M2e peptide or a peptide comprising an influenza hemagglutinin precursor protein cleavage site (HAO).

17. A pharmaceutical composition comprising the flavivirus of any of claims 11-16.

## Patentansprüche

1. Ein Verfahren zur Herstellung eines viralen Genoms, das ein Nukleinsäuremolekül umfasst, das ein heterologes, immunogenes Peptid kodiert, wobei das virale Genom das Genom eines Flaviviruses ist, und das Verfahren die Schritte umfasst:
(i) Zur Verfügung stellen eines viralen Zielgens;
(ii) Das virale Zielgen Mutagenese aussetzen, um eine Restriktionsschnittstelle einzufügen;
(iii) Ligieren eins Nukleinsäuremoleküls, das ein heterologes, immunogenes Peptid kodiert, in die Restriktionsschnittstelle des viralen Zielgens, um eine mutierte Gen-Bibliothek herzustellen;
(iv) Tranzfizieren von Zellen mit den viralen Genomen aus Schritt (iii), um Virusreplikation zu initiieren; und
(v) Selektieren lebensfähiger Virus-Rekombinanten mit einem Antikörper gegen das immunogene Peptid;
wobei das virale Zielgen zur Verfügung gestellt wird (a) in einem Shuttlevektor, und das Verfahren nach der Ligation des Nukleinsäuremoleküls, das das heterologe Peptide kodiert, in die Stelle der Mutagenese des viralen Zielgens, des Weiteren den Schritt des Einfügens des mutierten viralen Zielgens in ein virales Genom aus dem das virale Zielgen entstammt, an der Stelle des korrespondierenden viralen Gens ohne Insertion, umfasst, um virale Genome, die Insertionen umfassen, herzustellen, oder (b) im Zusammenhang eines intakten viralen Genoms, und das Verfahren virale Genome, die die Insertionen enthalten, herstellt.

2. Verfahren nach Anspruch 1, wobei der Shuttlevektor zwei oder mehrere virale Zielgene umfasst.

3. Verfahren nach Anspruch 1 oder 2, des Weiteren umfassend die Herstellung eines viralen Vektors aus dem viralen Genom, das eine Insertion umfasst, durch Einfügen des viralen Genoms, das eine Insertion umfasst, in Zellen.

4. Verfahren nach Anspruch 3, des Weiteren umfassend Isolierung des viralen Vektors aus den Zellen oder dem Überstand dieser.

5. Verfahren nach einem der Ansprüche 1 bis 4, wobei der Mutageneseschritt die Einfügung von einem oder mehreren Transprimern in das virale Zielgen durch Transposon Mutagenese umfasst.

6. Verfahren nach einem der Ansprüche 1 bis 5, des Weiteren umfassend die Herstellung einer Bibliothek von mutierten viralen Genen.

7. Verfahren nach Anspruch 1, wobei das virale Genom das Genom eines chimären Flavivirus ist.

8. Verfahren nach einem der Ansprüche 1 bis 7, wobei das virale Zielgen aus der Gruppe bestehend aus Genen, die Hüll-, Kapsid-, pre-Membran-, NS1-, NS2A-, NSB-, NS3-, NS4A-, NS4B-, und NS5-Proteine kodieren, ausgewählt ist.

9. Verfahren nach einem der Ansprüche 1 bis 8, wobei das heterologe, immunogene Peptid ein Impfepitop umfasst.

10. Verfahren nach Anspruch 9, wobei das Impfepitop ein B-Zell-Epitop oder ein T-Zell-Epitop ist.

11. Ein Flavivirus umfassend ein Genom, in welchem mindestens ein Nukleinsäuremolekül, das ein heterologes, immunogenes Peptid kodiert, innerhalb einer Sequenz, die ein Protein ausgewählt aus der Gruppe bestehend aus Pre-Membranprotein und dem Nichtstrukturellen Protein 1 (NS1) kodiert, eingefügt ist, wobei die Einfügung in NS1 zwischen den Sequenzen, die die Aminosäuren 236 und 237 davon kodieren, liegt, und/oder die Einfügung an einer Position -4, -2, oder -1 vor Sequenzen, die die Kapsid-pre-Membran Schnittstelle kodieren, oder Sequenzen, die Position 26 des pre-Membranproteins kodieren, liegt.

12. Flavivirus gemäß Anspruch 11, des Weiteren umfassend mindestens ein Nukleinsäuremolekül, das ein heterologes, immunogenes Peptid kodiert, das innerhalb einer Sequenz eingefügt ist, die ein Protein kodiert, das aus der Gruppe bestehend aus Kapsid, Hülle, NS2A, NS2B, NS3, NS4A, NS4B, und NS5 ausgewählt ist.

13. Flavivirus gemäß Anspruch 11, wobei das Flavivirus ein Gelbfiebervirus oder ein chimäres Flavivirus ist.

14. Verfahren nach Anspruch 7 oder Flavivirus nach Anspruch 13, wobei das Genom des chimären Flavivirus Sequenzen umfasst, die das Kapsid- und Nicht-strukturelle Protein eines ersten Flavivirus und die pre-Membran- und Hüllproteine eines zweiten, verschiedenen Flavivirus kodieren, umfasst.

15. Verfahren oder Flavivirus nach Anspruch 14, wobei der erste und der zweite Flavivirus unabhängig voneinander aus der Gruppe bestehend aus Japanische Enzephalitis, Dengue-1, Dengue-2, Dengue-3, Dengue-4, Gelbfieber, Murray Valley Enzephalitis, St. Louis Enzephalitis, West Nil, Kunjin, Rocio Enzephalitis, Ilheus, Frühsommer-Meningoenzephalitis, Zentraleuropäische Enzephalitis, Sibirische Enzephalitis, Russische Frühjahr-Sommer-Enzephalitis, Kyasanur-Wald-Fieber, Omsker Fieber, Louping ill-Enzephalitis, Powassan-Enzephalitis, Negishi, Absettarov, Hansalova, Apoi, und Hypr Viren, ausgewählt sind.

16. Verfahren nach einem der Ansprüche 1-9, 14 und 15 oder das Flavivirus nach einem der Ansprüche 11-15, wobei das heterologe Peptid ein Influenza-M2e-Peptid umfasst, oder ein Peptid, das eine Influenza-Hemagglutinin-Vorläuferprotein-Schnittstelle (HAO) umfasst.

17. Eine pharmazeutische Zusammensetzung, die das Flavivirus nach einem der Ansprüche 11-16 umfasst.

## Revendications

1. Procédé pour produire un génome viral comprenant une molécule d'acide nucléique codant pour un peptide immunogène hétérologue, dans lequel le génome viral est le génome d'un flavivirus, le procédé comprenant les étapes consistant à:
(i) procurer un gène viral cible;
(ii) soumettre le gène viral cible à une mutagenèse pour insérer un site de restriction;
(iii) ligaturer une molécule d'acide nucléique codant pour un peptide immunogène hétérologue, dans le site de restriction du gène viral cible pour produire une bibliothèque de gènes mutants;
(iv) transfecter les cellules avec les génomes viraux de l'étape (iii) pour initier la réplication du virus; et
(v) sélectionner des recombinants viraux viables avec un anticorps contre le peptide immunogène;
tandis que le gène viral cible est fourni (a) dans un vecteur navette et, après ligature de la molécule d'acide nucléique codant pour le peptide hétérologue dans le site de mutagenèse du gène viral cible, le procédé comprenant en outre l'étape d'introduction du gène viral cible muté dans un génome viral d'où a été dérivé le gène viral cible, au lieu du gène viral correspondant dénué de l'insertion, pour produire des génomes viraux comprenant des insertions, ou (b) dans le contexte d'un génome viral intact, et le procédé produit des génomes viraux comprenant des insertions.

2. Procédé selon la revendication 1, dans lequel le vecteur navette comprend deux gènes viraux cibles ou plus.

3. Procédé selon la revendication 1 ou 2, comprenant en outre la production d'un vecteur viral à partir du génome viral comprenant une insertion par introduction du génome viral comprenant une insertion dans des cellules.

4. Procédé selon la revendication 3, comprenant en outre l'isolement du vecteur viral à partir des cellules ou de leur surnageant.

5. Procédé selon l'une quelconque des revendications 1-4, dans lequel l'étape de mutagenèse comprend l'introduction d'une ou plusieurs trans-amorces dans le gène viral cible par mutagenèse par transposon.

6. Procédé selon l'une quelconque des revendications 1-5, comprenant en outre la production d'une bibliothèque de gènes viraux mutés.

7. Procédé selon la revendication 1, dans lequel le génome viral est le génome d'un flavivirus chimérique.

8. Procédé selon l'une quelconque des revendications 1-7, dans lequel le gène viral cible est choisi dans le groupe consistant en des gènes codant pour des protéines d'enveloppe, de capside, de pré-membrane, de NS1, NS2A, NSB, NS3, NS4A, NS4B, et NS5.

9. Procédé selon l'une quelconque des revendications 1-8, dans lequel le peptide immunogène hétérologue comprend un épitope vaccinal.

10. Procédé selon la revendication 9, dans lequel l'épitope vaccinal est un épitope de cellule B ou un épitope de cellule T.

11. Flavivirus comprenant un génome dans lequel au moins une molécule d'acide nucléique codant pour un peptide immunogène hétérologue est insérée dans une séquence codant pour une protéine choisie dans le groupe consistant en protéine de pré-membrane et protéine non-structurale 1 (NS1), tandis que l'insertion dans NS1 s'effectue entre les séquences codant pour ses acides aminés 236 et 237, et/ou l'insertion s'effectue sur la position -4, -2, ou -1 précédant les séquences codant pour le site de clivage de capside/pré-membrane, ou les séquences codant pour la position 26 de la protéine de pré-membrane.

12. Flavivirus selon la revendication 11, comprenant en outre au moins une molécule d'acide nucléique codant pour un peptide immunogène hétérologue inséré dans une séquence codant pour une protéine choisie dans le groupe consistant en capside, enveloppe, NS2A, NS2B, NS3, NS4A, NS4B et NS5.

13. Flavivirus selon la revendication 11, dans lequel le flavivirus est un virus de la fièvre jaune, ou un flavivirus chimérique.

14. Procédé selon la revendication 7 ou flavivirus selon la revendication 13, dans lequel le génome du flavivirus chimérique comprend des séquences codant pour les protéines de capside et non-structurales d'un premier flavivirus et les protéines de pré-membrane et d'enveloppe d'un deuxième flavivirus, différent.

15. Procédé ou flavivirus selon la revendication 14, dans lequel le premier et le deuxième flavivirus sont indépendamment choisis dans le groupe consistant en les virus d'encéphalite japonaise, Dengue-1, Dengue-2, Dengue-3, Dengue-4, fièvre jaune, encéphalite de la vallée de la Murray, encéphalite de Saint-Louis, West Nile, Kunjin, encéphalite de Rocio, Ilheus, encéphalite transmise par des tiques, encéphalite d'Europe centrale, encéphalite de Sibérie, encéphalite russe verno-estivale, maladie de la forêt de Kyasanur, fièvre hémorragique d'Omsk, maladie de Louping, Powassan, Negishi, Absettarov, Hansalova, Apoi, et Hypr.

16. Procédé selon l'une quelconque des revendications 1-9, 14 et 15 ou flavivirus selon l'une quelconque des revendications 11-15, dans lequel le peptide hétérologue comprend un peptide M2e de la grippe ou un peptide comprenant un site de clivage de la protéine précurseur d'hémagglutinine de la grippe (HAO).

17. Composition pharmaceutique comprenant le flavivirus selon l'une quelconque des revendications 11-16.
